# EUROPEAN PATENT APPLICATION

(11) **EP 3 939 988 A1**
(43) Date of publication of application: **19.01.2022**
(21) Application number: 20774643.9
(22) Date of filing: 20.03.2020
(51) Int. Cl.: C07H 23/00, C07H 19/167, C07D 493/04, A61P 35/00, A61K 31/357, A61K 31/7076

(54) **PHARMACEUTICAL COMPOSITION COMPRISING NOVEL AZOLOPYRIMIDINE HETEROCYCLIC COMPOUND AS ACTIVE INGREDIENT**

(30) Priority: 20.03.2019 KR 20190031613; 19.03.2020 KR 20200033564
(71) Applicant: Korea Research Institute of Chemical Technology, Daejeon 34114 (KR)
(72) Inventor: PARK, Seong Jun, Daejeon 34114 (KR); LEE, Chang Hoon, Daejeon 34114 (KR); LIM, Hwan Jung, Daejeon 34114 (KR)
(74) Representative: Frick, Robert
(86) International application number: PCT/KR2020/003827
(87) International publication number: WO 2020/190073

(57) **Abstract**

The present invention relates to a pharmaceutical composition for the prophylaxis or treatment of cancer comprising a novel azolopyrimidine heterocyclic compound as an active ingredient, and the pharmaceutical composition for the prophylaxis or treatment of cancer of the present invention can be used as a small molecular immunotherapy anticancer agent which modulates an adenosine pathway by comprising the azolopyrimidine heterocyclic compound.

## Description

### [Technical Field]

The present invention relates to a pharmaceutical composition for prophylaxis or treatment of cancer including a novel azolopyrimidine heterocyclic compound, a prodrug thereof, a hydrate thereof, a solvate thereof, a stereoisomer thereof, or a pharmaceutically acceptable salt thereof as an effective component.

### [Background Art]

Cancer is a mass of cells composed of undifferentiated cells which proliferate unlimitedly while ignoring a necessary state in a tissue unlike normal cells which may proliferate and be inhibited regularly in a controlled manner, and is also referred to as a tumor. Cancer cells which proliferate unlimitedly as such constantly penetrate surrounding tissues and spread to other organs of the body to cause serious pain, resulting in death. Cancer is one of incurable diseases to be solved by humankind, and it is demanded to develop an effective therapeutic agent for treating cancer.

Surgery or a chemical anticancer drug (cytotoxic chemotherapy) to inhibit cell proliferation is mainly used in treatment of cancer.

A chemical anticancer drug is a therapy to kill cancer cells using a chemical to kill fast-growing cells (cytotoxic anticancer drug); however, since a chemical anticancer drug is not a targeted therapeutic agent to directly act on a target where each cancer occurs, when a chemotherapy treatment is repeated, side effects due to cytotoxicity and drug resistance occur, and thus, in spite of an initial successful response to an anticancer drug used in chemotherapy, when a cancer-fighting period is prolonged or cancer recurs, the treatment eventually fails due to side effects due to cytotoxicity and drug resistance.

Thus, a targeted anticancer drug to attack only specific cancer cells has been developed, but the targeted anticancer drug still also damages normal cells and has limited targets, and most of all, the resistance of the drug easily occurs.

In order to supplement the problems of the chemotherapeutic agent and the targeted anticancer drug, an immune anticancer drug which activates immune cells in the body to block an immuno-suppressant, improves an immune function, and also treats cancer has been developed. Complex interactions between cancer cells and immune cells are the most important factor to determine the survival or death of initial cancer cells, and the immune cells detect genetic modification accumulated inside the cancer cells to attack the cancer cells, thereby preventing proliferation and metastasis of cancer cells. In order to prevent a reaction or overreaction to an autoantigen, there are various suppression checkpoint signals including programmed death 1 (PD-1), programmed death 2 (PD-2), cytotoxic T-lymphocyte-associated antigen 4 (CTLA-4), and adenosine. Purine nucleoside which is extracellular adenosine is produced during an acute inflammation process by conversion from adenosine triphosphate (ATP) by ectonucleotidase ecto-5'-nucleotidase (CD73) and ectonucleoside triphosphate diphosphohydrolase-1 (CD39) expressed on the surface of cells of various kinds of tissues, is usually present at a high concentration in a cancer tissue, and is an important mediator in alteration of immune cell function in cancer.

Adenosine is a nucleoside composed of adenine and D-ribose, and is, in particular, a resistance factor having a cell protection activity in cell damage conditions having a limited oxygen and substrate supply (for example, Ischemia in various organs such as heart and brain).

Action of adenosine is mediated by specific receptors. Specifically, adenosine receptors (Ars) are G protein-coupled receptors (GPCR) which are expressed a lot in various cells, and are present as a total of four subtypes of A1, A2A, A2B, and A3. The A2A and A2B receptors increase cyclic adenosine monophosphate (cAMP), while the A1 or A3 receptor decreases cAMP, and thus, intracellular signaling is influenced depending on the kind of expressed receptors.

Thus, there is a very high demand for development for a novel material which inhibits the enzyme activity of CD73 and CD39 to prevent conversion from ATP into adenosine, thereby preventing a cancer tissue-specific immunity lowering mechanism. Furthermore, since extracellular ATP induces various immune activities by receptors such as P2X7 present in immune cells, CD73 and CD39 inhibitors are very good targets to enhance anticancer immunity.

Therefore, it is demanded to study various compounds having inhibitory ability against CD73 and Cd39.

### [Disclosure]

### [Technical Problem]

Thus, the present inventors found that an azolopyrimidine heterocyclic compound having a specific structure inhibits an enzyme activity of CD73 and CD39 to prevent conversion from ATP into adenosine, thereby suppressing a cancer tissue-specific immunity lowering mechanism to enhance anticancer immunity so that proliferation and growth of cancer cells are inhibited, and completed the present invention.

An object of the present invention is to provide a pharmaceutical composition for prophylaxis or treatment of cancer including the azolopyrimidine heterocyclic compound, a prodrug thereof, a hydrate thereof, a solvate thereof, a stereoisomer thereof, or a pharmaceutically acceptable salt thereof as an effective component.

### [Technical Solution]

In one general aspect,

a pharmaceutical composition for prophylaxis or treatment of cancer includes: an azolopyrimidine heterocyclic compound represented by the following Chemical Formula 1, a prodrug thereof, a hydrate thereof, a solvate thereof, a stereoisomer thereof, or a pharmaceutically acceptable salt thereof as an effective component:

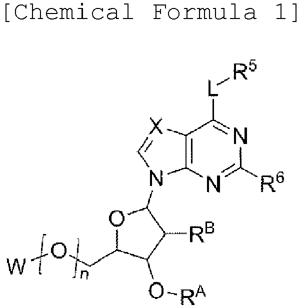

wherein
R^{A} is hydrogen, C1-C20alkyl, or acetyl;
R^{B} is hydrogen, a halogen, or -O-R^{c};
R^{c} is hydrogen, C1-C20alkyl, or acetyl, or the R^{c} may be linked to R^{A} via C1-C20alkylene to form a fused ring;
X is CR' or N;
R' is hydrogen, C1-C20alkyl, or C3-C20cycloalkyl;
L is a single bond, NR", O, S, or S=O;
R" is hydrogen, C1-C20alkyl, or C3-C20cycloalkyl;
n is an integer of 0 or 1;
W is 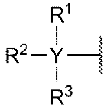 or 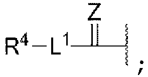
Y is C or Si;
R¹ is C1-C20alkyl, C6-C20aryl, C2-C20heteroaryl, or C2-C20heterocycloalkyl;
R² and R³ are independently of each other hydrogen, C1-C20alkyl, C6-C20aryl, C2-C20heteroaryl, or C2-C20heterocycloalkyl, or R² and R³ may be linked via C1-C20alkaylene to form a fused ring;
Z is O or S;
L¹ is a single bond, NR"', O, or S;
R‴ is hydrogen, C1-C20alkyl, or C3-C20cycloalkyl;
R⁴ is C6-C20aryl, C3-C20cycloalkyl, C2-C20heteroaryl, or C2-C20heterocycloalkyl;
R⁵ is hydrogen, a halogen, C1-C20alkyl, -(CH₂)ₘ-Ar¹, C6-C20aryl, C3-C20cycloalkyl, C2-C20heteroaryl, or C2-C20heterocycloalkyl;
m is an integer of 1 to 10;
Ar¹ is C6-C20aryl, C3-C20cycloalkyl, C2-C20heteroaryl, or C2-C20heterocycloalkyl;
R⁶ is hydrogen, a halogen, or NR^{a}R^{b};
R^{a} and R^{b} are independently of each other hydrogen, C1-C20alkyl, C3-C20cycloalkyl, or C6-C20aryl;
the alkyl, aryl, heteroaryl, or heterocycloalkyl of R¹ to R³, the alkyl, aryl, cycloalkyl, heteroaryl, or heterocycloalkyl of R⁵, the aryl, cycloalkyl, heteroaryl, or heterocycloalkyl of R⁴ and Ar¹, or the alkyl or aryl of R^{a} and R^{b} may be further substituted by one or more selected from a halogen, C1-C20alkyl, haloC1-C20alkyl, C1-C20alkoxy, haloC1-C20alkoxy, C6-C20aryl, C6-C20aryloxy, C1-C20alkylsulfanyl, haloC1-C20alkylsulfanyl, C3-C20cycloalkylsulfanyl, C6-C20arylsulfanyl, pentafluorosulfanyl, pentafluorosulfanyloxy, C3-C20cycloalkyl, C2-C20heteroaryl, C2-C20alkenyl, C2-C20alkynyl, amino, C1-C20alkylamino, C6-C20arylamino, hydroxy, C1-C20alkylcarbonyl, C1-C20alkoxycarbonyl, C1-C20alkylcarbonyloxy, C1-C20alkoxycarbonyloxy, nitro, mercapto, formyl, carboxyl, sulfamoyl, and carbamoyl; and
the heteroaryl and the heterocycloalkyl contain one or more heteroatom selected from nitrogen, oxygen, and sulfur.

### [Advantageous Effects]

The pharmaceutical composition for prophylaxis or treatment of cancer of the present invention includes an azolopyrimidine heterocyclic compound, and the azolopyrimidine heterocyclic compound inhibits an enzyme activity of CD73 and CD39 to prevent conversion from ATP into adenosine, thereby suppressing a cancer tissue-specific immunity-lowering mechanism to enhance anticancer immunity so that proliferation and growth of cancer cells may be significantly suppressed, and has low cytotoxicity to normal cells. Accordingly, the pharmaceutical composition for prophylaxis or treatment of cancer including the azolopyrimidine heterocyclic compound of the present invention may be very useful for prophylaxis and treatment of cancer.

### [Best Mode]

Hereinafter, the present invention will be described in detail. Technical terms and scientific terms used in the present specification have the general meaning understood by those skilled in the art to which the present invention pertains unless otherwise defined, and a description for the known function and configuration obscuring the present invention will be omitted in the following description.

The term "alkyl" in the present specification refers to a monovalent linear or branched saturated hydrocarbon radical composed of only carbon and hydrogen atoms. The alkyl may have 1 to 20 carbon atoms. The alkyl may have 1 to 10 carbon atoms. The alkyl may have 1 to 7 carbon atoms. The alkyl includes methyl, ethyl, propyl, isopropyl, butyl, isobutyl, t-butyl, pentyl, hexyl, and the like, but is not limited thereto.

The term "cycloalkyl" in the present specification is a monovalent saturated or unsaturated carbocyclic radical composed of one or more rings, and is not an aromatic group. The cycloalkyl may be monocyclic or a fused, spiro, or crosslinked bicyclic ring system. The cycloalkyl may have 3 to 20, preferably 3 to 10, and more preferably 3 to 7 carbon atoms. Specifically, a monocyclic cycloalkyl ring includes 3 to 10 carbon atoms, preferably 3 to 7 carbon atoms in a ring. Specifically, a bicyclic cycloalkyl ring includes 7 to 17 carbon atoms, preferably 1 to 12 carbon atoms in a ring. A preferred bicyclic cycloalkyl ring includes a 4-, 5-, 6-, or 7-membered ring fused to a 5-, 6-, or 7-membered ring. A specific example of cycloalkyl includes cyclopropyl, cyclobutyl, cyclopentyl, cyclohexyl, and the like, but is not limited thereto.

The term "aryl" in the present specification refers to a monovalent organic radical of an aromatic ring derived from aromatic hydrocarbon by removal of one hydrogen, including a single- or fused ring system containing appropriately 4 to 7, preferably 5 or 6 ring atoms in each ring, and even a form in which a plurality of aryls are connected by a single bond. A specific example thereof includes phenyl, naphthyl, biphenyl, anthryl, indenyl, fluorenyl, and the like, but is not limited thereto.

The term "halo" or "halogen" in the present specification refers to a halogen group element, and for example, includes fluoro, chloro, bromo, and iodo.

The term "alkoxy" in the present specification is an -O-alkyl radical, and may have 1 to 20 carbon atoms, preferably 1 to 10 carbon atoms, and more preferably 1 to 7 carbon atoms. Herein, "alkyl" is as defined above. A specific example thereof includes methoxy, ethoxy, isopropoxy, butoxy, isobutoxy, t-butoxy, and the like, but is not limited thereto.

The term "haloalkyl" described in the present specification refers to alkyl substituted by one or more halogens, and an example thereof may include trifluoromethyl and the like.

The term "haloalkoxy" described in the present specification refers to alkoxy substituted by one or more halogens, and an example thereof may include trifluoromethoxy and the like.

The term "heteroaryl" in the present specification refers to an aryl group containing 1 to 4 heteroatoms selected from N, O, and S as a ring skeleton atom and carbon as a remaining aromatic ring skeleton atom, and is 5- or 6-membered monocyclic heteroaryl and polycyclic heteroaryl fused with one or more benzene rings, which may be partially saturated. In addition, the heteroaryl in the present specification also includes a form in which one or more heteroaryls are connected by a single bond. An example of the heteroaryl includes pyrrolyl, quinolyl, isoquinolyl, pyridyl, pyrimidyl, oxazolyl, thiazolyl, thiadiazolyl, triazolyl, imidazolyl, benzoimidazolyl, isoxazolyl, benzoisoxazolyl, thienyl, benzothienyl, furyl, benzofuryl, and the like, but is not limited thereto.

The term "heterocycloalkyl" in the present specification is a monovalent radical of a non-aromatic heterocycle containing 1 to 4 heteroatoms selected from N, O, and S, and the non-aromatic heterocycle includes all forms of saturated or unsaturated monocycle, polycycle, and spirocycle, and may be bonded via a heteroatom or a carbon atom, and a nitrogen, carbon, oxygen, or sulfur atom in a non-aromatic heterocyclic radical are in various oxidation states and may be oxidized, if necessary. In addition, a nitrogen atom in the non-aromatic heterocyclic radical may be quaternarized. An example of the heterocycloalkyl radical may include monovalent radicals of non-aromatic heterocycles such as aziridine, pyrrolidine, pyrrolidinone, azetidine, piperidine, tetrahydropyridine, piperazine, morpholine, thiomorpholine, 3-azabicyclo[3.1.0]hexane, octahydropyrrolo[3,4-c]pyrrole, 2,7-diazispiro[4.4]nonane, 2-azaspiro[4.4]nonane, and the like.

The term "aryloxy" in the present specification refers to an -O-aryl radical, wherein "aryl" is as defined above. An example of the aryloxy radical includes phenoxy, and the like, but is not limited thereto.

The term "alkylsulfanyl" in the present specification refers to a -S-alkyl radical, wherein "alkyl" is as defined above. An example of the alkylsulfanyl radical includes methylsulfanyl, ethylsulfanyl, and the like, but is not limited thereto.

The term "cycloalkylsulfanyl" in the present specification refers to an -S-cycloalkyl radical, wherein "cycloalkyl" is as defined above. An example of the cycloalkylsulfanyl radical includes cyclopropylsulfanyl, cyclobutylsulfanyl, cyclopentylsulfanyl, cyclohexylsulfanyl, and the like, but is not limited thereto.

The term "arylsulfanyl" in the present specification refers to an -S-aryl radical, wherein "aryl" is as defined above. An example of the alkylsulfanyl radical includes phenylsulfanyl, naphthylsulfanyl, and the like, but is not limited thereto.

The term "alkylamino" in the present specification refers to an amino radical in which one or two alkyls are substituted, and a specific example thereof includes methylamino (-NHMe), dimethylamino (-NMe₂), and the like, but is not limited thereto.

The term "arylamino" in the present specification refers to an amino radical in which one or two aryls are substituted, and a specific example thereof includes phenylamino(-NHPh), diphenylamino(-NPh₂), and the like, but is not limited thereto.

The term "alkylcarbonyl" in the present specification refers to a -C(=O)alkyl radical, wherein "alkyl" is as defined above. An example of the alkylcarbonyl radical includes methylcarbonyl, ethylcarbonyl, isopropylcarbonyl, propylcarbonyl, butylcarbonyl, isobutylcarbonyl, t-butylcarbonyl, and the like, but is not limited thereto.

The term "alkoxycarbonyl" in the present specification refers to a -C(=O)alkoxy radical, wherein "alkoxy" is as described above. An example of the alkoxycarbonyl radical includes methoxycarbonyl, ethoxycarbonyl, isopropoxycarbonyl, propoxycarbonyl, butoxycarbonyl, isobutoxycarbonyl, t-butoxycarbonyl, and the like, but is not limited thereto.

The term "alkylcarbonyloxy" in the present specification refers to an -OC(=O)alkyl radical, wherein "alkyl" is as described above. An example of the alkylcarbonyloxy radical includes methylcarbonyloxy, ethylcarbonyloxy, isopropylcarbonyloxy, propylcarbonyloxy, butylcarbonyloxy, isobutylcarbonyloxy, t-butylcarbonyloxy, and the like, but is not limited thereto.

The term "alkoxycarbonyloxy" in the present specification refers to a -OC(=O)alkoxy radical, wherein "alkoxy" is as described above. An example of the alkoxycarbonyloxy radical includes methoxycarbonyloxy, ethoxycarbonyloxy, isopropoxycarbonyloxy, propoxycarbonyloxy, butoxycarbonyloxy, isobutoxycarbonyloxy, t-butoxycarbonyloxy, and the like, but is not limited thereto.

In the present specification, the term "pentafluorosulfanyl" refers to -SF₆, "pentafluorosulfanyloxy" refers to -OSF₅, "amino" refer to -NH₂, "hydroxy" refers to -OH, "nitro" refers to -NO₂, "mercapto" refers to -SH, "formyl" refers to -COH, "carboxyl" refers to -COOH, "sulfamoyl" refers to -SO₂NH₂, and "carbamoyl" refers to -CONH₂.

The present invention provides a pharmaceutical composition for prophylaxis or treatment of cancer including: an azolopyrimidine heterocyclic compound represented by the following Chemical Formula 1, a prodrug thereof, a hydrate thereof, a solvate thereof, a stereoisomer thereof, or a pharmaceutically acceptable salt thereof as an effective component:

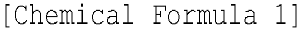

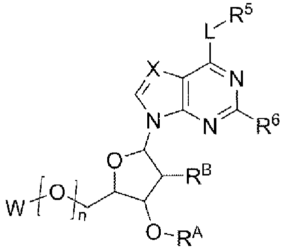

wherein
R^{A} is hydrogen, C1-C20alkyl, or acetyl;
R^{B} is hydrogen, a halogen, or -O-R^{c};
R^{c} is hydrogen, C1-C20alkyl, or acetyl, or the R^{c} may be linked to R^{A} via C1-C20alkylene to form a fused ring;
X is CR' or N;
R' is hydrogen, C1-C20alkyl, or C3-C20cycloalkyl;
L is a single bond, NR", O, S, or S=O;
R" is hydrogen, C1-C20alkyl, or C3-C20cycloalkyl;
n is an integer of 0 or 1;
W is 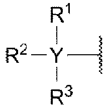 or 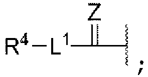
Y is C or Si;
R¹ is C1-C20alkyl, C6-C20aryl, C2-C20heteroaryl, or C2-C20heterocycloalkyl;
R² and R³ are independently of each other hydrogen, C1-C20alkyl, C6-C20aryl, C2-C20heteroaryl, or C2-C20heterocycloalkyl, or R² and R³ may be linked via C1-C20alkaylene to form a fused ring;
Z is O or S;
L¹ is a single bond, NR"', O, or S;
R‴ is hydrogen, C1-C20alkyl, or C3-C20cycloalkyl;
R⁴ is C6-C20aryl, C3-C20cycloalkyl, C2-C20heteroaryl, or C2-C20heterocycloalkyl;
R⁵ is hydrogen, a halogen, C1-C20alkyl, -(CH₂)ₘ-Ar¹, C6-C20aryl, C3-C20cycloalkyl, C2-C20heteroaryl, or C2-C20heterocycloalkyl;
m is an integer of 1 to 10;
Ar¹ is C6-C20aryl, C3-C20cycloalkyl, C2-C20heteroaryl, or C2-C20heterocycloalkyl;
R⁶ is hydrogen, a halogen, or NR^{a}R^{b};
R^{a} and R^{b} are independently of each other hydrogen, C1-C20alkyl, C3-C20cycloalkyl, or C6-C20aryl;
the alkyl, aryl, heteroaryl, or heterocycloalkyl of R¹ to R³, the alkyl, aryl, cycloalkyl, heteroaryl, or heterocycloalkyl of R⁵, the aryl, cycloalkyl, heteroaryl, or heterocycloalkyl of R⁴ and Ar¹, or the alkyl or aryl of R^{a} and R^{b} may be further substituted by one or more selected from a halogen, C1-C20alkyl, haloC1-C20alkyl, C1-C20alkoxy, haloC1-C20alkoxy, C6-C20aryl, C6-C20aryloxy, C1-C20alkylsulfanyl, haloC1-C20alkylsulfanyl, C3-C20cycloalkylsulfanyl, C6-C20arylsulfanyl, pentafluorosulfanyl, pentafluorosulfanyloxy, C3-C20cycloalkyl, C2-C20heteroaryl, C2-C20alkenyl, C2-C20alkynyl, amino, C1-C20alkylamino, C6-C20arylamino, hydroxy, C1-C20alkylcarbonyl, C1-C20alkoxycarbonyl, C1-C20alkylcarbonyloxy, C1-C20alkoxycarbonyloxy, nitro, mercapto, formyl, carboxyl, sulfamoyl, and carbamoyl; and
the heteroaryl and the heterocycloalkyl contain one or more heteroatom selected from nitrogen, oxygen, and sulfur.

The azolopyrimidine heterocyclic compound according to an exemplary embodiment of the present invention inhibits an enzyme activity of CD73 and CD39 to prevent conversion from ATP into adenosine, thereby suppressing a cancer tissue-specific immunity lowering mechanism to enhance anticancer immunity so that proliferation and growth of cancer cells may be significantly inhibited. In addition, the azolopyrimidine heterocyclic compound according to the present invention shows low toxicity to normal cells.

Therefore, a pharmaceutical composition including the azolopyrimidine heterocyclic compound as an effective component is very effective for prophylaxis and treatment of cancer.

The azolopyrimidine heterocyclic compound of Chemical Formula 1 may be, specifically, a compound represented by the following Chemical Formula 2, Chemical Formula 3, or Chemical Formula 4: 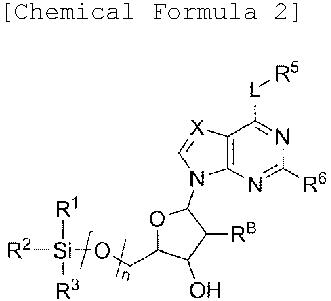 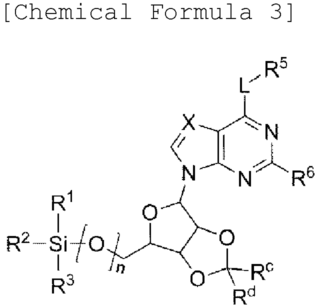 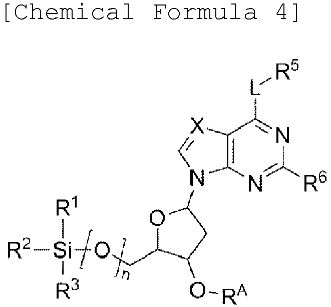
wherein X, L, R₁ to R₃, R₅, and R₆ are as defined in Chemical Formula 1;
n is an integer of 0 or 1;
R^{B} is hydrogen, a halogen, or hydroxy;
R^{A} is C1-C10alkyl; and
R^{c} and R^{d} are independently of each other hydrogen or C1-C10alkyl.

In Chemical Formulae 2 to 4, R¹ is C1-C10alkyl, C6-C12aryl, C2-C12heteroaryl, or C2-C10heterocycloalkyl; R² and R³ are independently of each other C1-C10alkyl, C6-C12aryl, C2-C12heteroaryl, or C2-C10heterocycloalkyl, or R² and R³ may be linked via C1-C10alkylene to form a fused ring; L is a single bond, NR", O, S, or S=O; R" is hydrogen or C1-C10alkyl; R⁵ is hydrogen, a halogen, C1-C10alkyl, - (CH₂)ₘ-Ar¹, C6-C12aryl, C3-C10cycloalkyl, C2-C12heteroaryl, or C2-C10heterocycloalkyl; m is an integer of 1 to 7; Ar¹ is C6-C12aryl, C3-C10cycloalkyl, C2-C12heteroaryl, or C2-C10heterocycloalkyl; R⁶ is hydrogen or a halogen; and the alkyl, aryl, heteroaryl, or heterocycloalkyl of R¹ to R³, the alkyl, aryl, cycloalkyl, heteroaryl, or heterocycloalkyl of R⁵, and the aryl, cycloalkyl, heteroaryl, or heterocycloalkyl of Ar¹ may be further substituted by one or more selected from a halogen, C1-C10alkyl, haloC1-C10alkyl, C1-C10alkoxy, haloC1-C10alkoxy, C6-C12aryl, C6-C12aryloxy, C1-C10alkylsulfanyl, haloC1-C10alkylsulfanyl, C3-C10cycloalkylsulfanyl, C6-C12arylsulfanyl, pentafluorosulfanyl, pentafluorosulfanyloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl.

The azolopyrimidine heterocyclic compound of Chemical Formula 1 may be, specifically, a compound represented by the following Chemical Formula 5, Chemical Formula 6, or Chemical Formula 7: 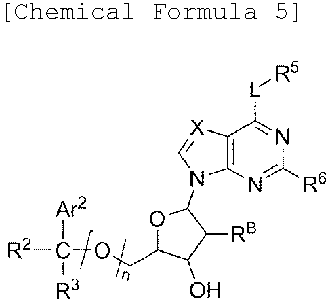 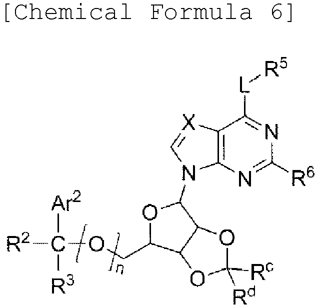 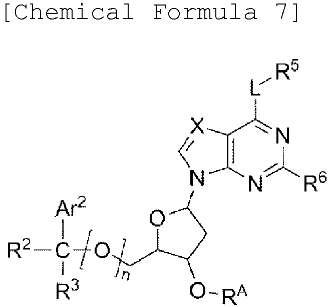
wherein X, L, R⁵, and R⁶ are as defined in Chemical Formula 1;
n is an integer of 0 or 1;
R^{B} is hydrogen, a halogen, or hydroxy;
R^{A} is C1-C10alkyl;
R^{c} and R^{d} are independently of each other hydrogen or C1-C10alkyl;
Ar² is C6-C12aryl or C2-C12heteroaryl;
R² and R³ are independently of each other hydrogen or C6-C12aryl; and
the aryl of Ar² or the aryl of R² and R³ may be further substituted by one or more selected from a halogen, C1-C10alkyl, C1-C10alkoxy, C6-C12aryl, C6-C12aryloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl, and the heteroaryl of Ar² may be further substituted by one or more selected from a halogen, C1-C10alkyl, haloC1-C10alkyl, C1-C10alkoxy, C6-C12aryl, C6-C12aryloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl.

The azolopyrimidine heterocyclic compound of Chemical Formula 1 may be, specifically, a compound represented by the following Chemical Formula 8, Chemical Formula 9, or Chemical Formula 10: 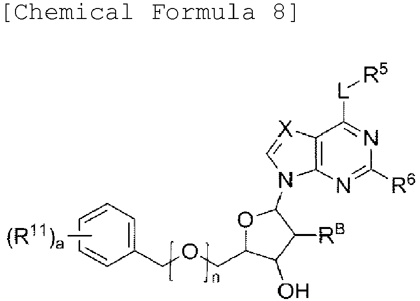 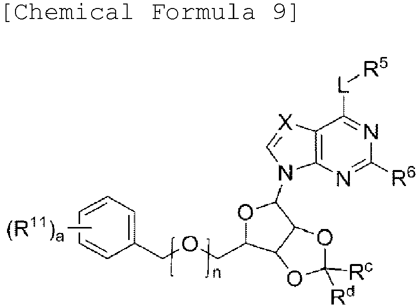 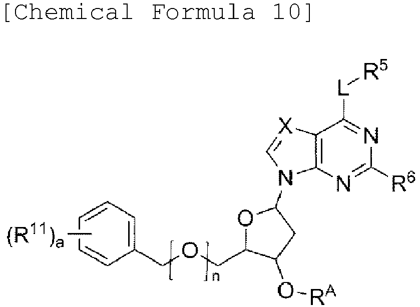
wherein X, L, R⁵, and R⁶ are as defined in Chemical Formula 1;
n is an integer of 0 or 1;
R^{B} is hydrogen, a halogen, or hydroxy;
R^{A} is C1-C10alkyl;
R^{c} and R^{d} are independently of each other hydrogen or C1-C10alkyl;
R¹¹ is haloC1-C10alkyl or pentafluorosulfanyl; and
a is an integer of 1 to 5.

The azolopyrimidine heterocyclic compound of Chemical Formula 1 may be, specifically, a compound represented by the following Chemical Formula 11, Chemical Formula 12, or Chemical Formula 13: [135] 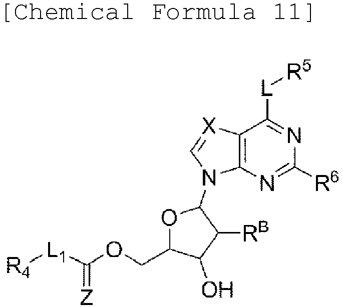 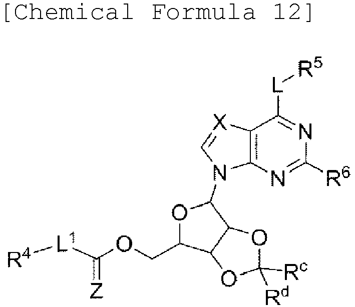 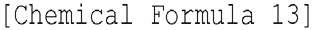 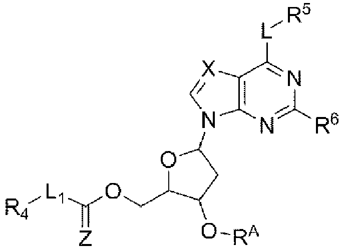
wherein X, L, R⁵, and R⁶ are as defined in Chemical Formula 1;
R^{B} is hydrogen, a halogen, or hydroxy;
R^{A} is C1-C10alkyl;
R^{c} and R^{d} are independently of each other hydrogen or C1-C10alkyl;
Z is O or S;
L¹ is a single bond, NH, or O;
R⁴ is C6-C12aryl or C2-C12heteroaryl; and
the aryl or heteroaryl of R⁴ may be further substituted by one or more selected from a halogen, C1-C10alkyl, haloC1-C10alkyl, C1-C10alkoxy, C6-C12aryl, C6-C12aryloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl.

In an exemplary embodiment, X may be N.

The azolopyrimidine heterocyclic compound of Chemical Formula 1 according to an exemplary embodiment may be, specifically, selected from the following compound group, but is not limited thereto:

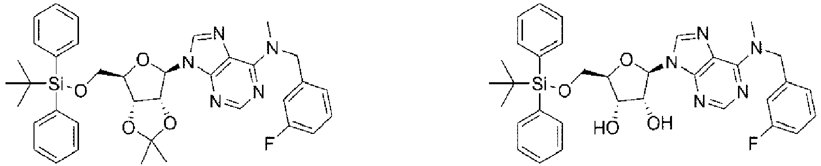

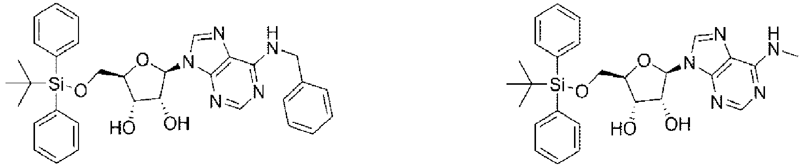

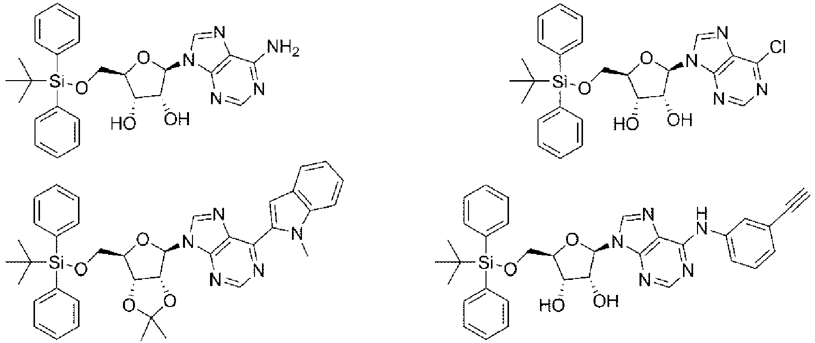

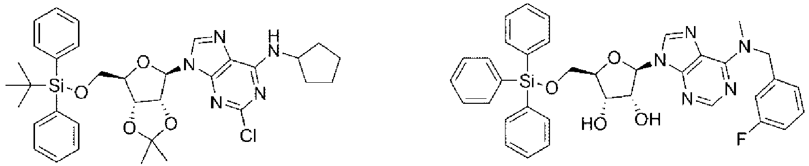

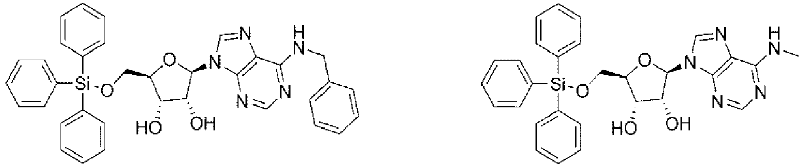

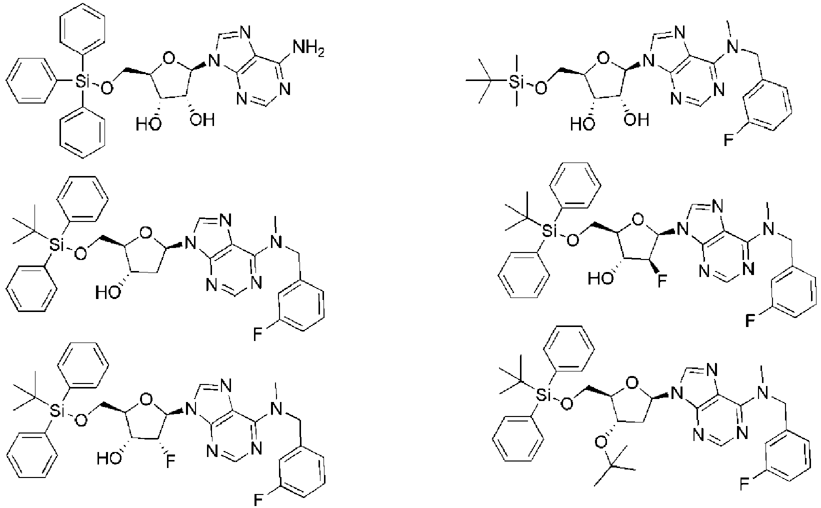

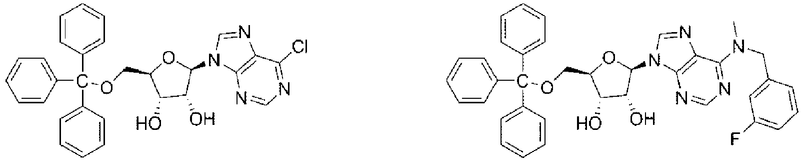

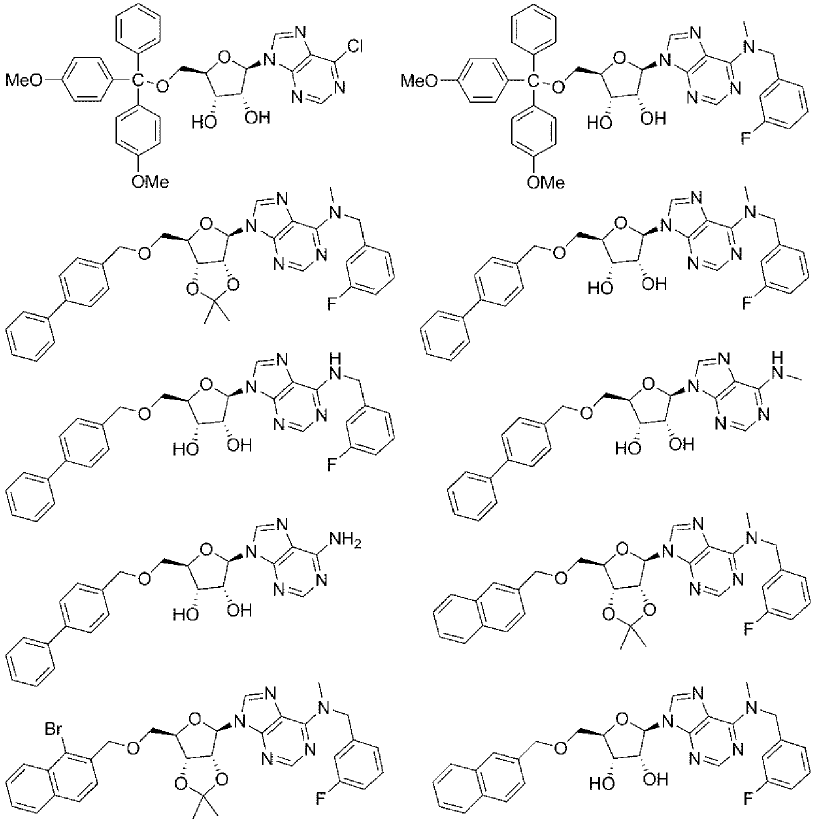

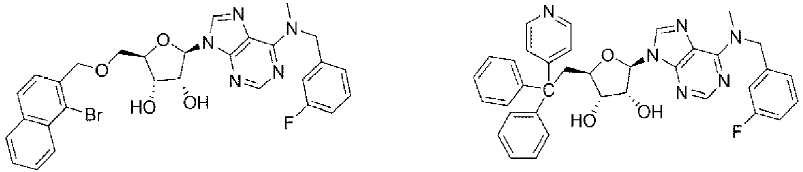

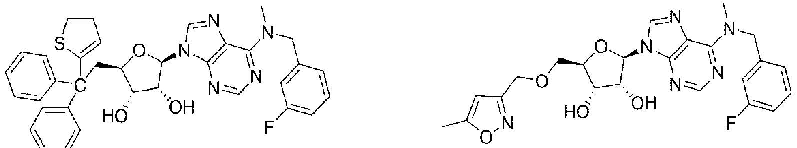

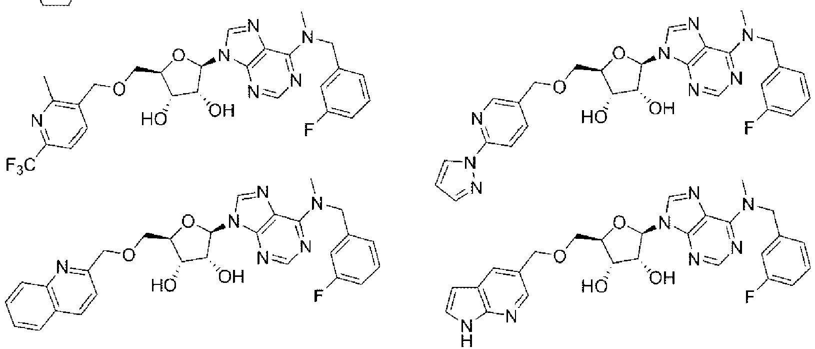

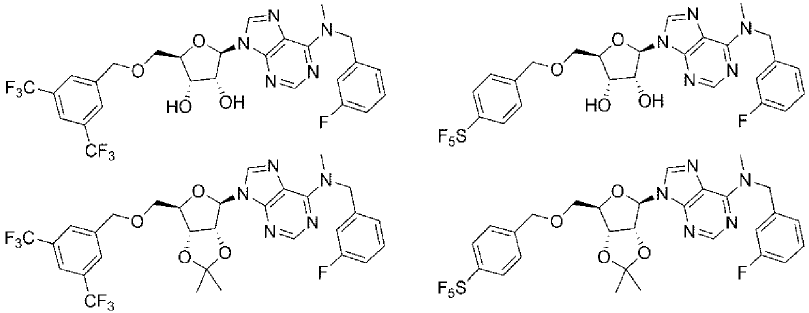

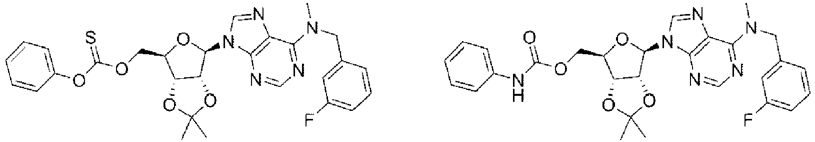

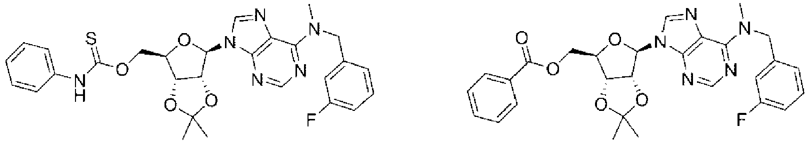

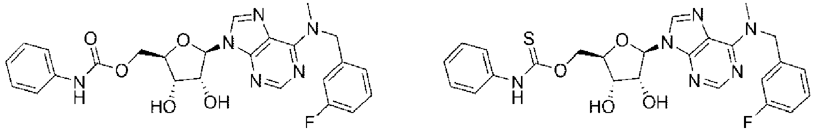

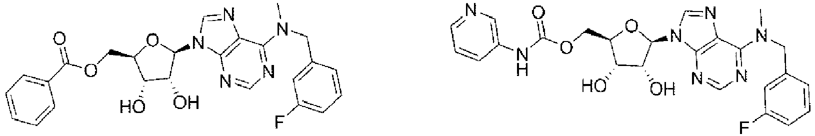

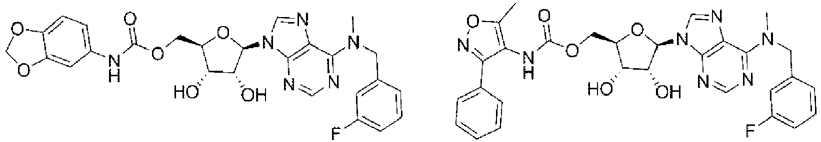

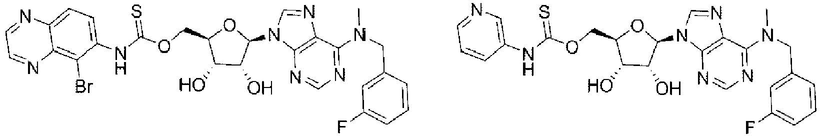

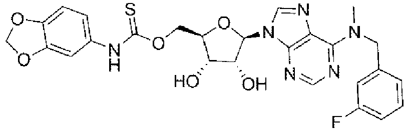

The azolopyrimidine heterocyclic compound of Chemical Formula 1 may be prepared by various methods known in the art depending on the kind of substituents, and it will be apparent to those skilled in the art that the preparation may be performed by using a method known or appropriately modifying the method.

Since the azolopyrimidine heterocyclic compound of the present invention may be used in the form of a prodrug, a hydrate, a solvate, and a pharmaceutically acceptable salt for enhancing in-vivo absorption or increasing solubility, the prodrug, the hydrate, the solvate, and the pharmaceutically acceptable salt also belong to the scope of the present invention. In addition, since the azolopyrimidine heterocyclic compound of the present invention has one or more chiral unsymmetric carbons, a stereoisomer thereof exists, and the stereoisomer also belongs to the scope of the present invention.

The azolopyrimidine heterocyclic compound of the present invention may be used in the form of a pharmaceutically acceptable salt. The "pharmaceutically acceptable salt" refers to any organic or inorganic addition salt of the compound of the present invention, in which the side effect caused by the salt does not reduce advantageous efficacy of the compound of the present invention itself at a concentration having a non-toxic and harmless effective action to a patient.

The pharmaceutically acceptable salt of the azolopyrimidine heterocyclic compound refers to a salt prepared according to a common method in the art, and the preparation method is known to those in the art. Specifically, the pharmaceutically acceptable salt includes pharmacologically or physiologically acceptable salts derived from the following inorganic acids, organic acid, and bases, but is not limited thereto.

The acid addition salt is prepared by a common method, for example, by dissolving the azolopyrimidine heterocyclic compound in an excessive amount of an aqueous acid solution and precipitating the salt using a water-compatible organic solvent, for example, methanol, ethanol, acetone, or acetonitrile. An equimolecular amount of the azolopyrimidine heterocyclic compound of the present invention and acid or alcohol (e.g., glycol monomethyl ether) in water are heated, and then the mixture may be dried by evaporation or the precipitated salt may be filtered by suction. Here, as a free acid, an organic acid and an inorganic acid may be used, and as the inorganic acid, hydrochloric acid, phosphoric acid, sulfuric acid, nitric acid, tartaric acid, and the like may be used and as the organic acid, methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, and the like, but the present invention is not limited thereto.

In addition, a pharmaceutically acceptable metal salt may be prepared using a base. An alkali metal salt or an alkali earth metal salt is obtained by, for example, dissolving the azolopyrimidine heterocyclic compound of the present invention in an excessive amount of an alkali metal hydroxide or alkali earth metal hydroxide solution, filtering the undissolved azolopyrimidine heterocyclic compound of the present invention, and then evaporating and drying a filtrate. Here, it is pharmaceutically appropriate to prepare particularly a sodium, potassium, or calcium salt as a metal salt, but the present invention is not limited thereto. In addition, a silver salt corresponding thereto may be obtained by reacting the alkali metal or alkali earth metal salt with an appropriate silver salt (e.g., silver nitrate).

The pharmaceutically acceptable salt of the azolopyrimidine heterocyclic compound of the present invention includes salts of an acidic or basic group which may be present in the azolopyrimidine heterocyclic compound of the present invention, unless otherwise indicated. For example, the pharmaceutically acceptable salt may include sodium, calcium, and potassium salts of a hydroxyl group, and other pharmaceutically acceptable salts of an amino group may include hydrobromate, sulfate, hydrogen sulfate, phosphate, hydrogen phosphate, dihydrogen phosphate, acetate, succinate, citrate, tartrate, lactate, mandelate, methanesulfonate (mesylate), p-toluenesulfonate (tosylate) salts, and the like, which may be prepared by a preparation method of salts known in the art.

A hydrate of the azolopyrimidine heterocyclic compound of the present invention refers to the azolopyrimidine heterocyclic compound of the present invention or the pharmaceutically acceptable salt thereof including a stoichiometric or non-stoichiometric amount of water bonded by a non-covalent intermolecular force.

A solvate of the azolopyrimidine heterocyclic compound of the present invention refers to the azolopyrimidine heterocyclic compound of the present invention or the pharmaceutically acceptable salt thereof including a stoichiometric or non-stoichiometric amount of a solvent bonded by a non-covalent intermolecular force. An available solvent includes a volatile and non-toxic solvent.

The azolopyrimidine heterocyclic compound of the present invention may have a chiral center and may exist as a racemate, a racemic mixture, and individual enantiomer or diastereomer. These isomers may be separated or resolved by a common method, and any predetermined isomer may be obtained by a common synthesis method or by stereospecific or asymmetric synthesis. These isomer forms and mixtures thereof are all included in the scope of the present invention.

The azolopyrimidine heterocyclic compound of the present invention may be administered in the form of a prodrug which is decomposed in a human or animal body to provide the compound of the present invention as an effective component. The prodrug may be used for modifying and/or improving a physical and/or pharmacokinetic profile of a parent compound, and may be formed when the parent compound contains an appropriate group or substituent which may be derived to form the prodrug.

Thus, the present invention provides a pharmaceutical composition for prophylaxis or treatment of cancer including: the azolopyrimidine heterocyclic compound represented by the Chemical Formula 1, the prodrug thereof, the hydrate thereof, the solvate thereof, the stereoisomer thereof, or the pharmaceutically acceptable salt thereof as an effective component.

The azolopyrimidine heterocyclic compound represented by the Chemical Formula 1, the prodrug thereof, the hydrate thereof, the solvate thereof, the stereoisomer thereof, or the pharmaceutically acceptable salt included in the pharmaceutical composition for prophylaxis or treatment of cancer may prevent or treat cancer as a low-molecular immune anticancer drug to regulate an adenosine pathway.

The term "prophylaxis" in the present specification refers to all actions to inhibit or delay the onset, spread and recurrence of cancer diseases by administering the composition of the present invention, and the term "treatment" refers to all actions to improve or change symptoms of cancer diseases advantageously.

The term "cancer" in the present specification refers to a cellular disorder characterized by unregulated or dysregulated cell proliferation, decreased cellular differentiation, inappropriate ability to invade surrounding tissues, and/or ability to establish new growth in an ectopic site.

Specifically, the cancer may be liver cancer, colon cancer, prostate cancer, stomach cancer, lung cancer or breast cancer.

In an exemplary embodiment, the pharmaceutical composition may further include a common, non-toxic, pharmaceutically acceptable carrier, excipient, or diluent in addition to the effective component, to be formulated into a common preparation in the pharmaceutical field, that is, a preparation for oral administration or a preparation for parenteral administration.

The term "pharmaceutically acceptable" in the present specification means that a characteristic of being non-toxic to an individual such as a cell or a human exposed to the composition is shown. The pharmaceutically acceptable carrier, excipient, or diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, and the like.

The pharmaceutical composition of the present invention may be formulated into various forms, for example, oral formulations such as powders, granules, tablets, capsules, suspensions, emulsions, syrups, and aerosols, injections of sterile injection solutions, and the like by a common method according to the purpose of use, and may be orally administered or administered by various routes including intravenous, intraperitoneal, subcutaneous, rectal, and topical administrations.

In addition, the pharmaceutical composition of the present invention may further include a filler, an anticoagulant, a lubricant, a wetting agent, a flavoring, an emulsifying agent, an antiseptic agent, and the like.

An example of a solid preparation for oral administration may include tablets, pills, powders, granules, capsules, and the like, and the solid preparation is formulated by mixing the composition with one or more excipients, for example, starch, calcium carbonate, sucrose, lactose, gelatin, and the like. In addition, in addition to a simple excipient, a lubricant such as magnesium stearate and talc is used.

An example of a liquid preparation for oral administration may include suspensions, oral liquids, emulsions, syrups, and the like, and various excipients, for example, a wetting agent, a sweetener, a flavoring agent, a preservative, and the like may be included in addition to water and liquid paraffin which are a commonly used simple diluent.

An example of a preparation for parenteral administration may include sterile aqueous solutions, nonaqueous solvent, suspensions, emulsions, freeze-dried preparations, suppositories, and the like. As the nonaqueous solvent and the suspension, propylene glycol, polyethylene glycol, a vegetable oil such as an olive oil, an injectable ester such as ethyloleate, and the like may be used. As a base of the suppository, witepsol, macrogol, tween 61, cacao butter, laurin butter, glycerogelatin, and the like may be used. Meanwhile, an injection may include a conventional additive such as a solubilizer, an isotonic agent, a suspending agent, an emulsifying agent, a stabilizer, and an antiseptic agent.

The pharmaceutical composition of the present invention may be sterilized, may further include an adjuvant such as an antiseptic agent, a stabilizer, a thickener, a hydrating agent or an emulsifying accelerator, a salt for regulating osmotic pressure, and/or a buffer, or may further include other therapeutically useful materials, and may be formulated according to a conventional method such as dissolving, dispersing, and gelling.

The pharmaceutical composition of the present invention is administered at a pharmaceutically effective amount. The term "pharmaceutically effective amount" in the present invention refers to an amount which is sufficient to treat a disease at a reasonable benefit/risk ratio applicable to medical treatment and does not cause side effects, and an effective dose level may be determined by factors including the health state of a patient, the kind of diseases, severity, an activity of a drug, a sensitivity to a drug, an administration manner, an administration time, administration route and releasing rate, a treatment period, and a simultaneously used drug, and other factors well known in the medical field.

The pharmaceutical composition of the present invention may be administered as an individual therapeutic agent or in combination with other therapeutic agents, may be administered sequentially or simultaneously with a conventional therapeutic agent, and may be administered in a single or multiple doses. It is important to administer an amount for obtaining a maximum effect with a minimum amount without any side effect, considering all of the above elements, and this will be easily determined by a person skilled in the art.

Specifically, the effective amount of the compound in the pharmaceutical composition of the present invention may be varied with the age, the gender, and the weight of a patent, and generally, 0.01 to 100 mg, preferably 1 to 50 mg per 1kg of a body weight may be administered daily or every other day or administered in a divided dose of one to several times a day. However, since the amount may be increased or decreased depending on an administration route, severity of the disease, gender, weight, age, and the like, the administration amount in no way limits the scope of the present invention.

Hereinafter, the present invention will be described in detail through the following preferred exemplary embodiments. However, this is presented by way of illustration of the present invention, and the right scope of the present invention is not limited thereby in any sense and the right scope of the present invention is only defined by the claims set forth below.

[Example 1] Preparation of 9- ((3a*R*,4*R*,6*R*,6a*R*)-6-(((tert-butyldiphenylsilyl)oxy)methyl)-2,2-dimethyltetrahydrofuro[3,4-*d*][1,3]dioxol-4-yl)-*N*-(3-fluorobenzyl)-*N*-methyl-9*H*-purin-6-amine (Compound 1)

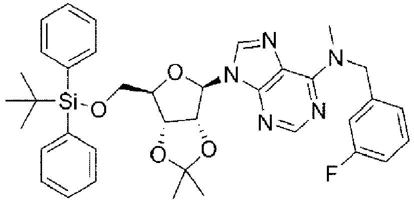

((3a*R*,4*R*,6*R*,6a*R*)-6-(6-((3-fluorophenyl)(methyl)amino)-9*H*-purin-9-yl)-2,2-dimethyltetrahydrofuro[3,4-d][1,3]dioxol-4-yl)methanol (50 mg, 0.116 mmol) was added to a 7 mL vial and was dissolved in DCM (0.1 M, 0.5 mL). Then, imidazole (8.7 mg, 1.1 eq, 0.128 mmol) was added at 0°C, the reactants were reacted for about 10 minutes, tert-butylchlorodiphenylsiliane (0.03 mL, 1.1 eq, 0.128 mmol) was added thereto, and the reactants were reacted at room temperature for 2 hours. After completion of the reaction, extraction with water (40 mL) and DCM (25 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (48.3 mg, yield: 61.5%).

¹H NMR (300 MHz, CDCl₃) δ 8.31 (s, 1H), 7.88 (s, 1H), 7.58 (ddt, J = 11.6, 6.6, 1.6 Hz, 4H), 7.46-7.21 (m, 6H), 7.27-7.23 (m, 1H), 7.11-6.84 (m, 3H), 6.13 (d, J = 2.6 Hz, 1H), 5.37 (s, 2H), 5.35 (dd, J = 6.2, 2.5 Hz, 1H), 4.97 (dd, J = 6.4, 2.8 Hz, 1H), 4.40 (td, J = 4.9, 2.7 Hz, 1H), 3.91 (dd, J = 11.1, 4.8 Hz, 1H), 3.78 (dd, J = 11.1, 5.1 Hz, 1H), 3.42 (s, 3H), 1.62 (s, 3H), 1.38 (s, 3H), 1.00 (s, 9H).

[Example 2] Preparation of *O*-(((3a*R*,4*R*,6*R*,6a*R*)-6-(6-((3-fluorobenzyl)(methyl)amino)-9*H*-purin-9-yl)-2,2-dimethyltetrahydrofuro[3,4-d][1,3]dioxol-4-yl)methyl) *O-*phenyl carbonothioate (Compound 2)

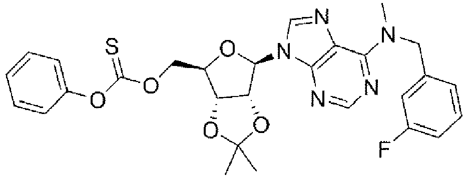

((3a*R*,4*R*,6*R*,6aR)-6-(6-((3-fluorophenyl)(methyl)amino)-9*H*-purin-9-yl)-2,2-dimethyltetrahydrofuro[3,4-d][1,3]dioxol-4-yl)methanol (50 mg, 0.116 mmol) and pyridine (0.02 mL, 2.0 eq, 0.232 mmol) were added to a 7 mL vial and were dissolved in DCM (6.0 M, 0.02 mL). Then, *O*-phenyl chlorothionoformate (0.02 mL, 1.1 eq, 0.128 mmol) was added thereto, and the reactants were reacted at room temperature for 2 hours. After completion of the reaction, extraction with water (40 mL) and DCM (25 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (44.6 mg, yield: 65.7%).

¹H NMR (300 MHz, CDCl₃) δ 8.39 (s, 1H), 7.88 (s, 1H), 7.39 (dd, J = 8.5, 6.9 Hz, 2H), 7.31-7.22 (m, 3H), 7.09-7.03 (m, 2H), 6.95 (ddt, J = 11.1, 8.6, 3.8 Hz, 2H), 6.19 (d, J = 2.1 Hz, 1H), 5.46 (dd, J = 6.2, 2.1 Hz, 1H), 5.32 (s, 2H), 5.20 (dd, J = 6.4, 2.8 Hz, 1H), 4.85 (dd, J = 10.5, 2.9 Hz, 1H), 4.75-4.65 (m, 2H), 3.41 (s, 3H), 1.65 (s, 3H), 1.42 (s, 3H).

[Example 3] Preparation of ((3a*R*,4*R*,6*R*,6a*R*)-6-(6-((3-fluorobenzyl)(methyl)amino)-9*H*-purin-9-yl)-2,2-dimethyltetrahydrofuro[3,4-d][1,3]dioxol-4-yl)methyl phenylcarbamate (Compound 3)

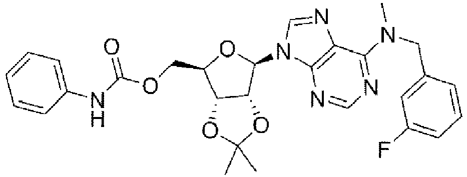

((3a*R*,4*R*,6*R*,6aR)-6-(6-((3-fluorophenyl)(methyl)amino)-9*H*-purin-9-yl)-2,2-dimethyltetrahydrofuro[3,4-d][1,3]dioxol-4-yl)methanol (64.7 mg, 0.15 mmol) and triethylamine (0.03 mL, 1.5 eq, 0.225 mmol) were added to a 7 mL vial and were dissolved in DCM (0.06 M, 2.5 mL). Then, phenyl isocyanate (0.04 ml, 2.2 eq, 0.33 mmol) was added, and the reactants were reacted at room temperature for 2 hours. After completion of the reaction, extraction with water (40 mL) and DCM (25 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (65.3 mg, yield: 81.4%).

¹H NMR (300 MHz, CDCl₃) δ 8.39 (s, 1H), 7.83 (s, 1H), 7.50-7.44 (m, 4H), 7.30-7.27 (m, 2H), 7.07-6.92 (m, 3H), 6.65 (s, 1H), 6.12 (d, J = 2.1 Hz, 1H), 5.57 (d, J = 6.2 Hz, 1H), 5.35 (s, 2H), 5.13 (dd, J = 6.3, 3.2 Hz, 1H), 4.50 (ddd, J = 15.8, 10.6, 4.6 Hz, 2H), 4.35 (dd, J = 11.3, 5.8 Hz, 1H), 3.40 (s, 3H), 1.62 (s, 3H), 1.41 (s, 3H).

[Example 4] Preparation of *O*-(((3a*R*,4*R*,6*R*,6a*R*)-6-(6-((3-fluorobenzyl)(methyl)amino)-9*H*-purin-9-yl)-2,2-dimethyltetrahydrofuro[3,4-d][1,3]dioxol-4-yl)methyl) phenylcarbamothioate (Compound 4)

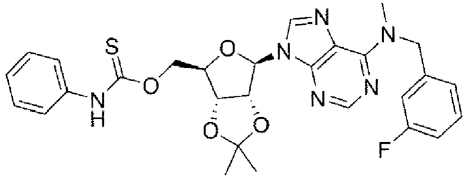

((3a*R*,4*R*,6*R*,6a*R*)-6-(6-((3-fluorophenyl)(methyl)amino)-9*H*-purin-9-yl)-2,2-dimethyltetrahydrofuro[3,4-d][1,3]dioxol-4-yl)methanol (60 mg, 0.153 mmol) was added to a 7 mL vial and was dissolved in THF (1.0 M, 0.15 mL). Then, sodium hydride (7 mg, 1.1 eq, 0.168 mmol, 60% in oil) was added at 0°C, the reactants were reacted for about 30 minutes, phenyl isothiocyanate (0.02 mL, 1.1 eq, 0.168 mmol) was added thereto, and the reactants were reacted at room temperature for 2 hours. After completion of the reaction, extraction with water (40 mL) and ethyl acetate (25 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (76.1 mg, yield: 90%).

¹H NMR (300 MHz, CDCl₃) δ 8.37 (s, 1H), 8.20 (s, 1H), 7.88-7.48 (m, 3H), 7.31-7.28 (m, 2H), 7.15 (s, 2H), 7.08-6.92 (m, 3H), 6.10 (d, J = 2.3 Hz, 1H), 5.66 (s, 1H), 5.12 (s, 2H), 4.73 (dd, J = 11.4, 5.6 Hz, 2H), 4.63 (t, J = 4.5 Hz, 2H), 3.40 (s, 3H), 1.63 (s, 3H), 1.41 (s, 3H).

[Example 5] Preparation of ((3a*R*,4*R*,6*R*,6a*R*)-6-(6-((3-fluorobenzyl)(methyl)amino)-9*H*-purin-9-yl)-2,2-dimethyltetrahydrofuro[3,4-d][1,3]dioxol-4-yl)methyl benzoate (Compound 5)

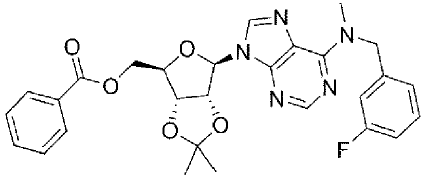

((3a*R*,4*R*,6*R*,6a*R*)-6-(6-((3-fluorophenyl)(methyl)amino)-9*H*-purin-9-yl)-2,2-dimethyltetrahydrofuro[3,4-d][1,3]dioxol-4-yl)methanol (50 mg, 0.116 mmol) and pyridine (0.02 mL, 2.0 eq, 0.232 mmol) were added to a 7 mL vial and were dissolved in DCM (6.0 M, 0.02 mL). Then, benzoyl chloride (0.015 mL, 1.1 eq, 0.128 mmol) was added thereto, and the reactants were reacted at room temperature for 2 hours. After completion of the reaction, extraction with water (40 mL) and DCM (25 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (41.1 mg, 65.9%).

¹H NMR (300 MHz, CDCl₃) δ 8.37 (s, 1H), 7.83 (s, 1H), 7.56-7.50 (m, 3H), 7.39-7.35 (m, 2H), 7.30-7.27 (m, 1H), 7.05-6.92 (m, 3H), 6.12 (d, J = 2.1 Hz, 2H), 5.59 (dd, J = 6.3, 2.2 Hz, 1H), 5.31 (s, 2H), 5.20-5.17 (m, 1H), 4.68-4.61 (m, 2H), 4.53-4.47 (m, 1H), 3.39 (s, 3H), 1.64 (s, 3H), 1.43 (s, 3H).

[Example 6] Preparation of ((2*R*,3*S*,4*R*,5*R*)-5-(6-((3-fluorobenzyl)(methyl)amino)-9*H*-purin-9-yl)-3,4-dihydroxytetrahydrofuran-2-yl)methyl phenylcarbamate (Compound 6)

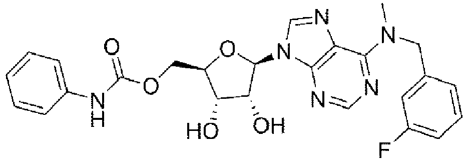

((3a*R*,4*R*,6*R*,6a*R*))-6-(6-((3-fluorobenzyl)(methyl)amino)-9*H*-purin-9-yl)-2,2-dimethyltetrahydrofuro[3,4-d][1,3]dioxol-4-yl)methyl phenylcarbamate (11 mg, 0.02 mmol) was added to a 7 mL vial and was dissolved in methanol (0.1 M, 0.2 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (5.7 mg, 56%) was obtained by recrystallization (Hex:DCM).

¹H NMR (300 MHz, MeOD-d₄) δ 8.26 (s, 1H), 8.19 (s, 1H), 7.38 (d, J = 8.0 Hz, 2H), 7.31 (t, J = 7.2 Hz, 1H), 7.24 (t, *J* = 8.0 Hz, 2H), 7.07 (d, J = 7.6 Hz, 1H), 6.98 (q, J = 8.5, 7.5 Hz, 3H), 6.06 (d, J = 4.9 Hz, 1H), 5.31 (s, 2H), 4.72 (t, J = 5.1 Hz, 1H), 4.52 (dd, J = 11.9, 3.4 Hz, 1H), 4.43-4.32 (m, 2H), 4.29 (q, J = 4.3 Hz, 1H), 3.37 (s, 3H).

[Example 7] Preparation of *O-(((2R,3S,4R,SR)-S-(6-*((3-fluorobenzyl)(methyl)amino)-9*H*-purin-9-yl)-3,4-dihydroxytetrahydrofuran-2-yl)methyl) phenylcarbamothioate (Compound 7)

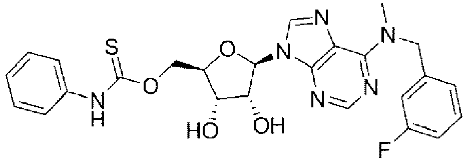

*O*-(((3a*R*,4*R*,6*R*,6a*R*)-6-(6-((3-fluorophenyl)(methyl)amino)-9*H*-purin-9-yl)-2,2-dimethyltetrahydrofuro[3,4-d][1,3]dioxol-4-yl)methyl) *O-*phenyl carbonothioate (11 mg, 0.019 mmol) was added to a 7 mL vial and was dissolved in methanol (0.1 M, 0.19 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (9.0 mg, 90%) was obtained by recrystallization (Hex:DCM).

¹H NMR (300 MHz, MeOD-d₄) δ 8.28 (s, 1H), 8.24 (s, 1H), 7.34-7.21 (m, 4H), 7.08 (d, J = 7.8 Hz, 2H), 7.05-6.93 (m, 4H), 6.07 (d, J = 3.8 Hz, 1H), 5.34 (s, 2H), 4.80-4.71 (m, 1H), 4.62 (s, 1H), 4.48-4.32 (m, 3H), 3.39 (s, 3H).

[Example 8] Preparation of (2*R,*3*S,*4*R,*5*R)-*2-(((*tert-*butyldiphenylsilyl)oxy)methyl)-5-(6-((3-fluorobenzyl)(methyl)amino)-9H-purin-9-yl)tetrahydrofuran-3,4-diol (Compound 8)

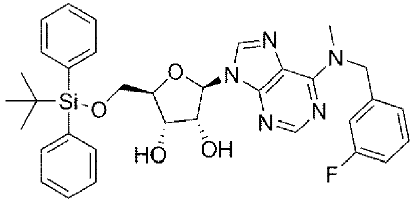

(2*R*,3*R*,4*S*,5*R*)-2-(6-(N-(3-fluorobenzyl)-N-methylamino)-9H-purin-9-yl)-5-(hydroxymethyl)tetrahydrofuran-3,4-diol (100 mg, 0.257 mmol) and 4-dimethylaminopyridine (3.1 mg, 0.1 eq, 0.026 mmol) were added to a 7 mL vial, and DMF (0.2 M, 0.5 mL) and pyridine (0.2 M, 0.5 mL) were added thereto and dissolution was performed. Then, tert-butylchlorodiphenylsilane (0.074 mL, 1.1 eq, 0.285 mmol) was added thereto, and the reactants were reacted at room temperature for 24 hours. After completion of the reaction, extraction with NH₄Cl (20 mL) and ethyl acetate (25 mL×2) was performed, and then an organic layer was dried with MgSO₄ and concentrated under reduced pressure. Thereafter, purification was performed with silica gel column chromatography (EA:Hex = 1:1 (v:v)) to obtain a title compound which is a white solid (119.3 mg, yield: 74%).

¹H NMR (300 MHz, CDCl₃) δ 8.31 (s, 1H), 7.99 (s, 1H), 7.54 (ddt, J = 9.7, 6.6, 1.5 Hz, 4H), 7.44-7.27 (m, 6H), 7.08-6.93 (m, 3H), 5.92 (d, J = 6.4 Hz, 1H), 5.30 (s, 2H), 4.64 (dd, J = 6.5, 5.0 Hz, 1H), 4.49 (dd, J = 5.0, 1.4 Hz, 1H), 4.46-4.40 (m, 1H), 3.87-3.76 (m, 2H), 3.18 (s, 3H), 0.89 (s, 9H).

[Example 9] Preparation of ((2*R,*3*S,*4*R,*5*R)-*5-(6-((3-fluorobenzyl)(methyl)amino)-9*H*-purin-9-yl)-3,4-dihydroxytetrahydrofuran-2-yl)methyl benzoate (Compound 9)

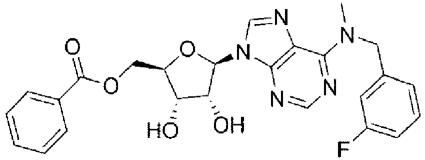

((3a*R*,4*R*,6*R*,6a*R*)-6-(6-((3-fluorobenzyl)(methyl)amino)-9*H*-purin-9-yl)-2,2-dimethyltetrahydrofuro[3,4-d] [1,3]dioxol-4-yl)methyl benzoate (10.7 mg, 0.02 mmol) was added to a 7 mL vial and was dissolved in methanol (0.1 M, 0.2 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (7.9 mg, 80%) was obtained by recrystallization (DCM:Hex).

¹H NMR (300 MHz, MeOD-d₄) δ 8.19 (s, 1H), 8.10 (s, 1H), 7.61-7.51 (m, 2H), 7.51-7.38 (m, 3H), 7.33-7.26 (m, 1H), 7.06 (d, J = 7.7 Hz, 1H), 7.03-6.92 (m, 2H), 6.03 (d, J = 4.3 Hz, 1H), 5.30 (s, 2H), 4.84 (t, J = 4.7 Hz, 1H), 4.70 (dd, J = 12.2, 3.5 Hz, 1H), 4.61-4.50 (m, 2H), 4.36 (td, J = 5.0, 3.5 Hz, 1H), 3.37 (s, 3H).

[Example 10] Preparation of 9- ((3a*R*,4*R*,6*R*,6a*R*)-6-(([1,1'-biphenyl]-4-ylmethoxy)methyl)-2,2-dimethyltetrahydrofuro[3,4-*d*][1,3]dioxol-4-yl)-*N*-(3-fluorobenzyl)-*N*-methyl-9*H*-purin-6-amine (Compound 10)

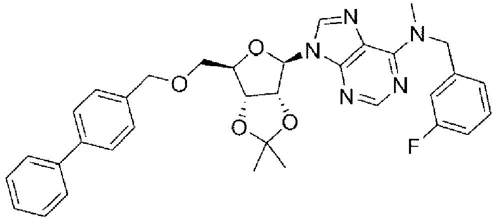

((3a*R*,4*R*,6*R*,6aR)-6-(6-((3-fluorobenzyl)(methyl)amino)-9*H*-purin-9-yl)-2,2-dimethyltetrahydrofuro[3,4-d][1,3]dioxol-4-yl)methanol (50 mg, 0.1164 mmol) was added to a 7 mL vial and was dissolved in DMF (0.1 M, 1.1 mL). Then, sodium hydride (9 mg, 2.0 eq, 0.23 mmol, 60% in oil) was added at 0°C, the reactants were reacted for about 30 minutes, 4-bromomethylbiphenyl (31.6 mg, 1.1 eq, 0.128 mmol) was added thereto, and the reactants were reacted at 80°C for 16 hours. After completion of the reaction, extraction with water (40 mL) and ethyl acetate (25 mL×2) was performed, and then an organic layer was dried with MgSO₄ and concentrated under reduced pressure. Thereafter, purification was performed with silica gel column chromatography (EA:Hex = 1:2) to obtain a title compound which is a sticky transparent liquid (60.4 mg, yield: 87.1%).

¹H NMR (300 MHz, CDCl₃) δ 8.37 (s, 1H), 7.95 (s, 1H), 7.65-7.39 (m, 7H), 7.37-7.31 (m, 1H), 7.27-7.21 (m, 3H), 7.09-6.86 (m, 3H), 6.19 (d, J = 2.6 Hz, 1H), 5.42-5.36 (m, 1H), 5.24 (s, 2H), 5.01 (dd, J = 6.2, 2.6 Hz, 2H), 4.61-4.49 (m, 3H), 3.75-3.66 (m, 2H), 3.37 (s, 3H), 1.64 (s, 3H), 1.40 (s, 3H).

[Example 11] Preparation of 9-((3a*R*,4*R*,6*R*,6a*R*)-6-(((3,5-bis(trifluoromethyl)benzyl)oxy)methyl)-2,2-dimethyltetrahydrofuro[3,4-*d*][1,3]dioxol-4-yl)-*N*-(3-fluorobenzyl)-*N*-methyl-9*H*-purin-6-amine (Compound 11)

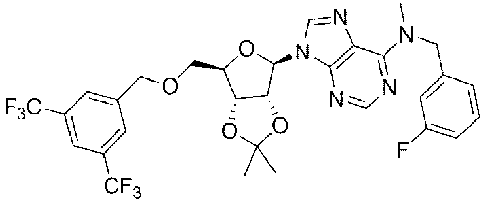

((3a*R*,4*R*,6*R*,6a*R*)-6-(6-((3-fluorobenzyl)(methyl)amino)-9*H*-purin-9-yl)-2,2-dimethyltetrahydrofuro[3,4-d][1,3]dioxol-4-yl)methanol (50 mg, 0.1164 mmol) was added to a 7 mL vial and was dissolved in DMF (0.1 M, 1.1 mL). Then, sodium hydride (9 mg, 2.0 eq, 0.23 mmol, 60% in oil) was added at 0°C, the reactants were reacted for about 30 minutes, 3,5-bis(trifluoromethyl)benzyl bromide (0.03 mL, 1.1 eq, 0.128 mmol) was added thereto, and the reactants were reacted at 80°C for 16 hours. After completion of the reaction, extraction with water (40 mL) and ethyl acetate (25 mL×2) was performed, and then an organic layer was dried with MgSO₄ and concentrated under reduced pressure. Thereafter, purification was performed with silica gel column chromatography (EA:Hex = 1:2) to obtain a title compound which is a sticky transparent liquid (57.5 mg, yield: 75.4%) .

¹H NMR (300 MHz, CDCl₃) δ 8.36 (s, 1H), 7.88 (s, 1H), 7.64 (d, J = 8.8 Hz, 2H), 7.28-7.23 (m, 3H), 7.07-6.91 (m, 3H), 6.16 (d, J = 2.1 Hz, 1H), 5.42-5.39 (m, 1H), 5.32 (s, 2H), 5.04 (dd, J = 6.3, 3.1 Hz, 2H), 4.53 (s, 2H), 4.52-4.48 (m, 1H), 3.72 (dd, J = 7.3, 4.4 Hz, 2H), 3.39 (s, 6H), 1.63 (s, 3H), 1.41 (s, 3H).

[Example 12] Preparation of 9-((3a*R*,4*R*,6*R*,6a*R*)-2,2-dimethyl-6-(((4-(pentafluorothio)benzyl)oxy)methyl)tetrahydrofuro[3,4-*d*][1,3]dioxol-4-yl)-*N*-(3-fluorobenzyl)-*N*-methyl-9*H*-purin-6-amine (Compound 12)

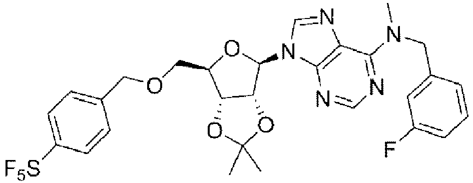

((3a*R*,4*R*,6*R*,6aR)-6-(6-((3-fluorobenzyl)(methyl)amino)-9*H*-purin-9-yl)-2,2-dimethyltetrahydrofuro[3,4-d][1,3]dioxol-4-yl)methanol (50 mg, 0.1164 mmol) was added to a 7 mL vial and was dissolved in DMF (0.1 M, 1.1 mL). Then, sodium hydride (9 mg, 2.0 eq, 0.23 mmol, 60% in oil) was added at 0°C, the reactants were reacted for about 30 minutes, 4-(pentafluorothio)benzyl bromide (38 mL, 1.1 eq, 0.128 mmol) was added thereto, and the reactants were reacted at 80°C for 16 hours. After completion of the reaction, extraction with water (40 mL) and ethyl acetate (25 mL×2) was performed, and then an organic layer was dried with MgSO₄ and concentrated under reduced pressure. Thereafter, purification was performed with silica gel column chromatography (EA:Hex = 1:2) to obtain a title compound which is a sticky transparent compound (46.5 mg, yield: 61.9%).

¹H NMR (300 MHz, CDCl₃) δ 8.33 (s, 1H), 7.84 (s, 1H), 7.77 (s, 1H), 7.70 (s, 2H), 7.28-7.25 (m, 1H), 7.08-6.91 (m, 3H), 6.14 (d, *J* = 2.3 Hz, 1H), 5.41 (dd, *J* = 6.3, 2.2 Hz, 1H), 5.30 (s, 2H), 5.10-5.06 (m, 1H), 4.60 (s, 2H), 4.54-4.48 (m, 1H), 3.82-3.75 (m, 2H), 3.40 (s, 3H), 1.63 (s, 3H), 1.41 (s, 3H).

[Example 13] Preparation of (2*R*,3*S*,4*R*,5*R*)-2-(([1,1'-biphenyl]-4-ylmethoxy)methyl)-5-(6-((3-fluorobenzyl)(methyl)amino)-9H-purin-9-yl)tetrahydrofuran-3,4-diol (Compound 13)

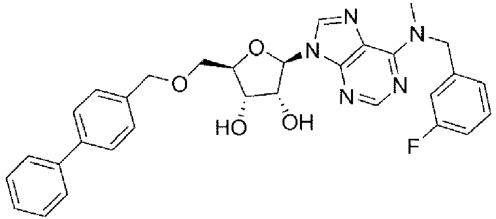

9-((3a*R*, 4*R*, 6*R*, 6a*R*) -6- (([1,1'-biphenyl]-4-ylmethoxy)methyl)-2,2-dimethyltetrahydrofuro[3,4-*d*][1,3]dioxol-4-yl)-*N*-(3-fluorobenzyl)-*N*-methyl-9*H*-purin-6-amine (10 mg, 0.017 mmol) was added to a 7 mL vial and was dissolved in methanol (0.1 M, 0.17 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (10 mg, yield: 99%) was obtained by recrystallization (DCM:Hex).

¹H NMR (300 MHz, CDCl₃) δ 8.30 (s, 1H), 8.00 (s, 1H), 7.58-7.53 (m, 2H), 7.50-7.39 (m, 5H), 7.37-7.33 (m, 1H), 7.21 (d, *J* = 8.2 Hz, 2H), 7.07-6.91 (m, 3H), 5.95 (d, J = 6.2 Hz, 1H), 5.36 (s, 2H), 4.60 (d, *J* = 5.0 Hz, 1H), 4.53-4.50 (m, 3H), 4.44 (dd, *J* = 5.0, 1.8 Hz, 1H), 3.70 (t, *J* = 3.8 Hz, 2H), 3.36 (s, 3H).

[Example 14] Preparation of (2*R*,3*S*,4*R*,5*R*)-2-(((3,5-bis(trifluoromethyl)benzyl)oxy)methyl)-5-(6-((3-fluorobenzyl)(methyl)amino)-9*H*-purin-9-yl) tetrahydrofuran-3,4-diol (Compound 14)

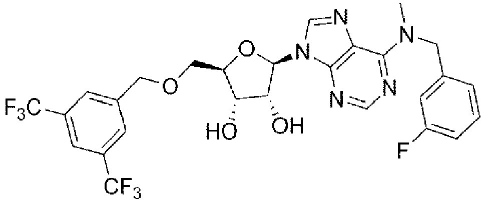

9-((3a*R*,4*R*,6*R*,6a*R*)-6-(((3,5-bis(trifluoromethyl)benzyl)oxy)methyl)-2,2-dimethyltetrahydrofuro[3,4-*d*][1,3]dioxol-4-yl)-*N*-(3-fluorobenzyl)-*N*-methyl-9*H*-purin-6-amine (11.5 mg, 0.0175 mmol) was added to a 7 mL vial and was dissolved in methanol (0.1 M, 0.18 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (10.8 mg, yield: 99%) was obtained by recrystallization (DCM:Hex).

¹H NMR (300 MHz, CDCl₃) δ 8.30 (s, 1H), 7.96 (s, 1H), 7.76 (s, 1H), 7.62 (s, 2H), 7.32-7.24 (m, 1H), 7.09-6.94 (m, 3H), 5.94 (d, *J* = 5.9 Hz, 1H), 5.32 (s, 2H), 4.62 (s, 2H), 4.55-4.51 (m, 2H), 4.45 (dd, *J* = 5.1, 1.9 Hz, 1H), 3.77 (dd, *J* = 3.7, 2.2 Hz, 2H), 3.36 (s, 3H).

[Example 15] Preparation of (2*R,*3*R*,4*S*,5*R)*-2-(6-((3-fluorobenzyl)(methyl)amino)-9*H*-purin-9-yl)-5-(((4-(pentafluorothio)benzyl)oxy)methyl)tetrahydrofuran-3,4-diol

### (Compound 15)

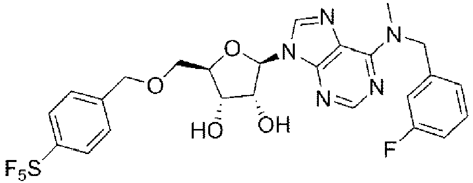

9-((3a*R*, 4*R*, 6*R*, 6a*R*) -2,2-dimethyl-6-(((4-(pentafluorothio)benzyl)oxy)methyl)tetrahydrofuro[3,4-*d*] [1,3]dioxol-4-yl)-*N*-(3-fluorobenzyl)-*N*-methyl-9*H*-purin-6-amine (11.5 mg, 0.0155 mmol) was added to a 7 mL vial and was dissolved in methanol (0.1 M, 0.16 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (9.9 mg, yield: 99%) was obtained by recrystallization (DCM:Hex).

¹H NMR (300 MHz, CDCl₃) δ 8.30 (s, 1H), 7.97 (s, 1H), 7.62 (d, *J* = 8.8 Hz, 2H), 7.32-7.24 (m, 1H), 7.20 (d, *J* = 8.2 Hz, 2H), 7.09-6.94 (m, 3H), 5.94 (d, *J* = 5.9 Hz, 1H), 5.39 (s, 2H), 4.57-4.50 (m, *J* = 8.2 Hz, 5H), 4.44 (dd, *J* = 5.1, 1.9 Hz, 2H), 3.71 (t, *J* = 3.4 Hz, 2H), 3.35 (s, 3H).

[Example 16] Preparation of 9-((3a*R*, 4*R*, 6*R*,6a*R*)-2,2-dimethyl-6-((naphthalen-2-ylmethoxy)methyl)tetrahydrofuro[3,4-*d*] [1,3]dioxol-4-yl)-*N-*(3-fluorobenzyl)-*N*-methyl-9*H*-purin-6-amine (Compound 16)

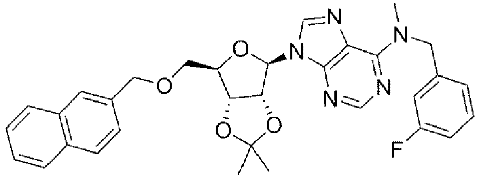

((3a*R*, 4*R*, 6*R*,6a*R*)-6-(6-((3-fluorobenzyl)(methyl)amino)-9*H*-purin-9-yl)-2,2-dimethyltetrahydrofuro[3,4-d][1,3]dioxol-4-yl)methanol (50 mg, 0.1164 mmol) was added to a 7 mL vial and was dissolved in DMF (0.1 M, 1.1 mL). Then, sodium hydride (9 mg, 2.0 eq, 0.23 mmol, 60% in oil) was added at 0°C, the reactants were reacted for about 30 minutes, 2-(bromomethyl)naphthalene (28.3 mg, 1.1 eq, 0.128 mmol) was added thereto, and the reactants were reacted at 80°C for 16 hours. After completion of the reaction, extraction with water (40 mL) and ethyl acetate (25 mL×2) was performed, and then an organic layer was dried with MgSO₄ and concentrated under reduced pressure. Thereafter, purification was performed with silica gel column chromatography (EA:Hex = 1:2) to obtain a title compound which is a sticky liquid (53.5 mg, yield: 81%).

¹H NMR (300 MHz, CDCl₃) δ 8.35 (s, 1H), 7.95 (s, 1H), 7.83-7.70 (m, 3H), 7.61 (s, 1H), 7.48-7.43 (m, 1H), 7.31 (dd, *J* = 8.3, 1.7 Hz, 1H), 7.28-7.21 (m, 1H), 7.04-6.90 (m, 3H), 6.19 (d, *J* = 2.6 Hz, 1H), 5.37 (dd, *J* = 6.2, 2.4 Hz, 1H), 5.22 (s, 2H), 5.01 (dd, *J* = 6.2, 2.6 Hz, 1H), 4.65 (s, 2H), 4.54 (q, *J* = 4.0, 3.5 Hz, 1), 3.72 (qd, *J* = 10.5, 4.2 Hz, 2), 3.35 (s, 3), 1.63 (s, 3), 1.40 (s, 3).

[Example 17] Preparation of 9-((3a*R*, 4*R*, 6*R*,6a*R*)-6-(((1-bromonaphthalen-2-yl)methoxy)methyl)-2,2-dimethyltetrahydrofuro[3,4-*d*][1,3]dioxol-4-yl)-*N*-(3-fluorobenzyl)-*N*-methyl-9*H*-purin-6-amine (Compound 17)

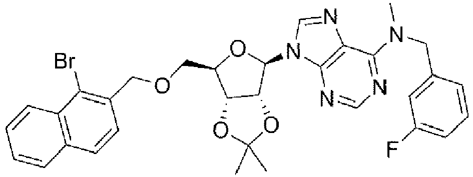

((3a*R*, 4*R*, 6*R*,6a*R*)-6-(6-((3-fluorobenzyl)(methyl)amino)-9H-purin-9-yl)-2,2-dimethyltetrahydrofuro[3,4-d][1,3]dioxol-4-yl)methanol (50 mg, 0.1164 mmol) was added to a 7 mL vial and was dissolved in DMF (0.1 M, 1.1 mL). Then, sodium hydride (9 mg, 2.0 eq, 0.23 mmol, 60% in oil) was added at 0°C, the reactants were reacted for about 30 minutes, 1-bromo-2-(bromomethyl)naphthalene (38.4 mg, 1.1 eq, 0.128 mmol) was added thereto, and the reactants were reacted at 80°C for 16 hours. After completion of the reaction, extraction with water (40 mL) and ethyl acetate (25 mL×2) was performed, and then an organic layer was dried with MgSO₄ and concentrated under reduced pressure. Thereafter, purification was performed with silica gel column chromatography (EA:Hex = 1:2) to obtain a title compound which is a sticky liquid (48.4 mg, yield: 64%).

¹H NMR (300 MHz, CDCl₃) δ 8.35 (s, 1H), 8.29 (d, *J* = 8.6 Hz, 1H), 7.93 (s, 1H), 7.81-7.75 (m, 1H), 7.65 (d, *J* = 8.6 Hz, 1H), 7.59 (ddd, *J* = 8.4, 6.8, 1.4 Hz, 1H), 7.51 (ddd, *J* = 8.1, 6.8, 1.4 Hz, 1H), 7.31 (d, *J* = 8.4 Hz, 1H), 7.27-7.20 (m, 1H), 7.02-6.90 (m, 3H), 6.19 (d, *J* = 2.4 Hz, 1H), 5.43 (dd, *J* = 6.2, 2.4 Hz, 1H), 5.23 (s, 2H), 5.06 (dd, *J* = 6.2, 2.6 Hz, 1H), 4.79 (s, 2H), 4.58 (q, *J* = 3.9, 3.3 Hz, 1H), 3.88-3.76 (m, 2H), 3.32 (s, 3H), 1.64 (s, 3H), 1.41 (s, 3H).

[Example 18] Preparation of (2*R*,3*R*,4*S*,5*R*)-2-(6-((3-fluorobenzyl)(methyl)amino)-9H-purin-9-yl)-5-((naphthalen-2-ylmethoxy)methyl)tetrahydrofuran-3,4-diol (Compound 18)

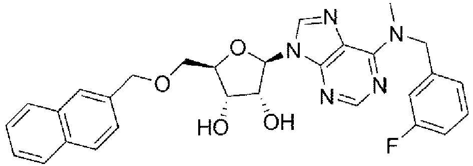

9-((3a*R*, 4*R*, 6*R*,6a*R*)-2,2-dimethyl-6-((naphthalen-2-ylmethoxy)methyl)tetrahydrofuro[3,4-*d*][1,3]dioxol-4-yl)-*N-*(3-fluorobenzyl)-*N*-methyl-9*H*-purin-6-amine (12.5 mg, 0.0168 mmol) was added to a 7 mL vial and was dissolved in methanol (0.1 M, 0.17 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (11.9 mg, yield: 99%) was obtained by recrystallization (DCM:Hex).

¹H NMR (300 MHz, CDCl₃) δ 8.41 (s, 1H), 7.90 (s, 1H), 7.86-7.71 (m, 4H), 7.50-7.46 (m, 3H), 7.30-7.28 (m, 1H), 7.08 (d, *J* = 7.9 Hz, 1H), 7.03-6.96 (m, 2H), 5.97 (d, *J* = 5.9 Hz, 1H), 5.44 (s, 2H), 4.88 (s, 1H), 4.77 (s, 2H), 4.73 (d, *J* = 5.0 Hz, 1H), 4.66 (s, 1H), 4.31-4.26 (m, 1H), 4.16 (t, *J* = 5.6 Hz, 1H), 3.73-3.66 (m, 2H), 3.50 (s, 3H).

[Example 19] Preparation of (2*R*,3*S*,4*R*,5*R*)-2-(((1-bromonaphthalen-2-yl)methoxy)methyl)-5-(6-((3-fluorobenzyl)(methyl)amino)-9H-purin-9-yl)tetrahydrofuran-3,4-diol (Compound 19)

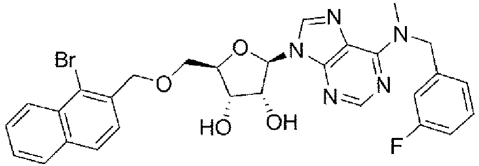

9-((3a*R*, 4*R*, 6*R*,6a*R*)-6-(((1-bromonaphthalen-2-yl)methoxy)methyl)-2,2-dimethyltetrahydrofuro[3,4-*d*] [1,3]dioxol-4-yl)-*N*-(3-fluorobenzyl)-*N*-methyl-9*H*-purin-6-amine (11.5 mg, 0.0175 mmol) was added to a 7 mL vial and was dissolved in methanol (0.1 M, 0.18 mL). Then, an aqueous hydrochloric acid solution (1 M, 2 mL) was added thereto, and the reactants were reacted at room temperature for 12 hours. After completion of the reaction, extraction with NaHCO₃ (10 mL) and ethyl acetate (15 mL×2) was performed, and then an organic layer was dried with MgSO₄ and concentrated under reduced pressure. Thereafter, a title compound which is a white solid (10.5 mg, yield: 99%) was obtained by recrystallization (DCM:Hex).

¹H NMR (300 MHz, CDCl₃) δ 8.39 (s, 1H), 8.28 (d, *J* = 8.2 Hz, 2H), 8.01 (s, 1H), 7.88 (s, 1H), 7.76 (d, *J* = 7.6 Hz, 2H), 7.66-7.48 (m, 3H), 7.30-7.27 (m, 1H), 7.09-6.98 (m, 3H), 5.96 (d, *J* = 6.2 Hz, 1H), 5.39 (s, 2H), 4.92 (s, 1H), 4.88 (d, *J* = 2.9 Hz, 1H), 4.82 (s, 2H), 4.62 (t, *J* = 5.6 Hz, 1H), 4.54 (s, 1H), 4.50-4.47 (m, 1H), 4.35-4.32 (m, 1H), 4.20-4.17 (m, 1H), 3.83-3.78 (m, 2H), 3.37 (s, 3H).

[Example 20] Preparation of (2*R*,3*S*,4*R*,5*R*)-2-(((*tert*butyldimethylsilyl)oxy)methyl)-5-(6-((3-fluorobenzyl)(methyl)amino)-9H-purin-9-yl)tetrahydrofuran-3,4-diol (Compound 20)

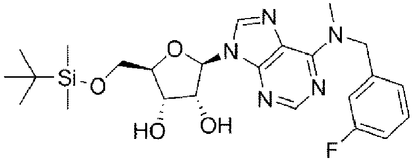

(2*R*,3*R*,4*S*,5*R*)-2-(6-((3-fluorobenzyl) (methyl) amino) - 9*H*-purin-9-yl)-5-(hydroxymethyl)tetrahydrofuran-3,4-diol (20 mg, 0.051 mmol) was added to a 7 mL vial and was dissolved in DCM (0.2 M, 0.3 mL). Then, imidazole (5.2 mg, 1.5 eq, 0.077 mmol) was added at 0°C, the reactants were reacted for about 10 minutes, *tert-*butylchlorodimethylsilane (9 mL, 1.2 eq, 0.061 mmol) was added thereto, and the reactants were reacted at room temperature for 3 hours. After completion of the reaction, extraction with water (10 mL) and DCM (15 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (10.0 mg, yield: 38.9%).

¹H NMR (300 MHz, CDCl₃) δ 8.28 (s, 1H), 8.09 (s, 1H), 7.30-7.23 (m, 1H), 7.10-6.92 (m, 3H), 6.08 (d, *J* = 5.0 Hz, 1H), 5.32 (s, 2H), 4.52 (t, *J* = 5.0 Hz, 1H), 4.42 (dd, *J* = 5.1, 3.1 Hz, 1H), 4.33 (q, *J* = 3.1 Hz, 1H), 3.95-3.81 (m, 4H), 3.43 (s, 3H), 0.82 (s, 9H), 0.05 (s, 6H).

[Example 21] Preparation of (2*R*,3*R*,4*S*,5*R)-*2-(6-((3-fluorobenzyl)(methyl)amino)-9*H*-purin-9-yl)-5-(((triphenylsilyl)oxy)methyl)tetrahydrofuran-3,4-diol

### (Compound 21)

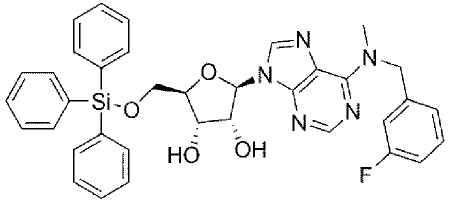

(2*R*,3*R*,4*S*,5*R*)-2-(6-(N-(3-fluorobenzyl)-N-methylamino)-9H-purin-9-yl)-5-(hydroxymethyl)tetrahydrofuran-3,4-diol (50 mg, 0.128 mmol) was added to a 7 mL vial and was dissolved in benzene (0.1 M, 1.3 mL). Then, 4-methylbenzenesulfonic acid monohydrate (2.5 mg, 0.1 eq, 0.013 mmol) and triphenylsilanol (35.4 mg, 1.0 eq, 0.128 mmol) were added thereto and the reactants were reacted by reflux for 12 hours. After completion of the reaction, extraction with water (20 mL) and ethyl acetate (25 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (3.4 mg, yield: 4.1%).

¹H NMR (300 MHz, CDCl₃) δ 8.26 (s, 1H), 7.81 (s, 1H), 7.67 (dd, *J* = 7.8, 1.9 Hz, 6H), 7.55-7.39 (m, 9H), 7.31-7.29 (m, 1H), 7.07-6.93 (m, 3H), 5.84 (d, *J* = 7.7 Hz, 1H), 5.35 (s, 2H), 5.04 (s, 1H), 4.78 (d, *J* = 5.6 Hz, 1H), 4.10 (s, 1H), 3.65 (d, *J* = 14.6 Hz, 1H), 3.37 (s, 3H), 3.09 (d,

*J* = 13.6 Hz, 1H), 2.92 (d, *J* = 10.1 Hz, 2H).

[Example 22] Preparation of (2*R*,3*R*,4*S*,5*R*)-2-(6-chloro-9*H*-purin-9-yl)-5-((trityloxy)methyl)tetrahydrofuran-3,4-diol (Compound 22)

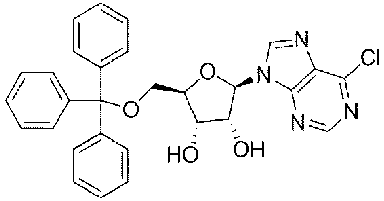

6-Chloropurine riboside (100 mg, 0.349 mmol), (chloromethanetriyl)tribenzene (194 mg, 2.0 eq, 0.697 mmol), DMF (0.1 M, 3.5 mL), and N,N-diisopropylethylamine (0.07 mL, 1.5 eq, 0.52 mmol) were added to a 7 mL vial and were reacted at 40°C for 24 hours. After completion of the reaction, extraction with water (20 mL) and ethyl acetate (15 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (97.9 mg, yield: 53%).

¹H NMR (300 MHz, CDCl₃) δ 8.68 (s, 1H), 8.36 (s, 1H), 7.32 (dd, *J* = 6.6, 3.0 Hz, 6H), 7.22 (dd, *J* = 5.0, 2.1 Hz, 9H), 6.08 (d, *J* = 5.4 Hz, 1H), 5.10 (d, *J* = 4.3 Hz, 1H), 4.87 (q, *J* = 4.7 Hz, 1H), 4.49 (dt, *J* = 5.0, 2.8 Hz, 1H), 4.39 (q, *J* = 3.3 Hz, 1H), 3.55 (d, *J* = 11.1 Hz, 1H), 3.49-3.34 (m, 2H).

[Example 23] Preparation of (2*R*,3*S*,4*R,*5*R*)-2-((bis(4-methoxyphenyl)(phenyl)methoxy)methyl)-5-(6-chloro-9H-purin-9-yl)tetrahydrofuran-3,4-diol (Compound 23)

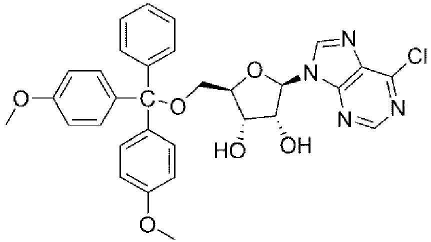

6-Chloropurine riboside (100 mg, 0.349 mmol), 4,4'-(chloro(phenyl)methylene)bis(methoxybenzene) (236.1 mg, 2.0 eq, 0.697 mmol), DMF (0.1 M, 3.5 mL), and *N,N-*diisopropylethylamine (0.07 mL, 1.5 eq, 0.52 mmol) were added to a 7 mL vial and were reacted at 40°C for 24 hours. After completion of the reaction, extraction with water (20 mL) and ethyl acetate (15 mL×2) was performed, and then an organic layer was dried with MgSO₄ and concentrated under reduced pressure. Thereafter, purification was performed with silica gel column chromatography (EA:Hex = 1:1) to obtain a title compound which is a red solid (49.4 mg, yield: 24%).

¹H NMR (300 MHz, CDCl₃) δ 8.73 (s, 1H), 8.38 (s, 1H), 7.24 (d, *J* = 3.2 Hz, 2H), 7.19-7.15 (m, 7H), 6.78-6.70 (m, 4H), 6.05 (d, *J* = 5.5 Hz, 1H), 4.99 (s, 1H), 4.88 (s, 1H), 4.45 (d, *J* = 10.3 Hz, 2H), 3.77 (s, 6H), 3.45 (dd, *J* = 10.8, 3.4 Hz, 1H), 3.35-3.32 (dd, *J* = 10.8, 3.2 Hz, 1H), 3.04 (s, 1H) .

[Example 24] Preparation of (2*R*,3*R*,4*S*,5*R*)-2-(6-((3-fluorobenzyl)(methyl)amino)-9*H*-purin-9-yl)-5-((trityloxy)methyl)tetrahydrofuran-3,4-diol (Compound 24)

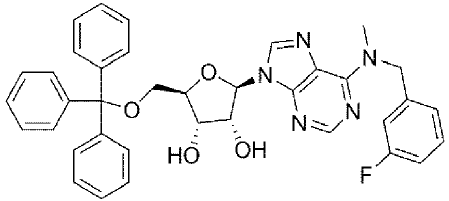

(2*R*,3*R*,4*S*,*5*R)-2-(6-chloro-9*H*-purin-9-yl)-5-((trityloxy)methyl)tetrahydrofuran-3,4-diol (96.9 mg, 0.183 mmol), 3-fluoro-*N*-methylbenzylamine (0.02 mL, 1.1 eq, 0.2 mmol), n-Butanol (0.6 M, 0.3 mL), and *N*,*N-*diisopropylethylamine (0.06 mL, 2.0 eq, 0.366 mmol) were added to a 7 mL vial and were reacted at 80°C for 3 hours. After completion of the reaction, extraction with water (20 mL) and ethyl acetate (15 mL×2) was performed, and then an organic layer was dried with MgSO₄ and concentrated under reduced pressure. Thereafter, purification was performed with silica gel column chromatography (MeOH:DCM = 1:10) to obtain a title compound which is a yellow solid (104.1 mg, yield: 90%).

¹H NMR (300 MHz, CDCl₃) δ 8.29 (s, 1H), 8.04 (s, 1H), 7.32 (dd, *J* = 7.6, 2.3 Hz, 6H), 7.26-7.14 (m, 10H), 7.06-6.91 (m, 3H), 5.98 (d, *J* = 5.4 Hz, 1H), 5.34 (s, 2H), 4.69 (d, *J* = 5.1 Hz, 1H), 4.38 (ddd, *J* = 8.1, 5.6, 3.0 Hz, 2H), 3.62 (t, *J* = 6.6 Hz, 1H), 3.47 (dd, *J* = 10.5, 3.5 Hz, 1H), 3.40 (s, 3H), 3.25 (dd, *J* = 10.6, 3.6 Hz, 1H).

[Example 25] Preparation of (2*R*,3*S*,4*R*,5*R*)-2-((bis(4-methoxyphenyl)(phenyl)methoxy)methyl)-5-(6-((3-fluorobenzyl)(methyl)amino)-9H-purin-9-yl)tetrahydrofuran-3,4-diol (Compound 25)

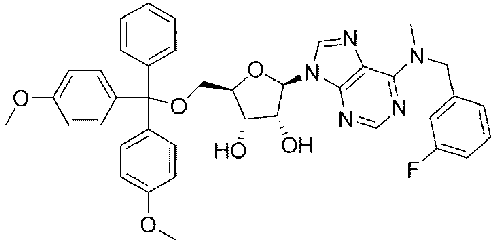

(2*R*,3*R*,*4S,*5*R*)-2-((bis(4-methoxyphenyl)(phenyl)methoxy)methyl)-5-(6-chloro-9H-purin-9-yl)tetrahydrofuran-3,4-diol (48.4 mg, 0.08 mmol) and 3-fluoro-*N*-methylbenzylamine (0.012 mL, 1.1 eq, 0.09 mmol), and then n-butanol (0.6 M, 0.3 mL) and *N*,*N-*diisopropylethylamine (0.03 mL, 2.0 eq, 0.16 mmol) were added to a 7 mL vial and were reacted at 80°C for 3 hours. After completion of the reaction, extraction with water (20 mL) and ethyl acetate (15 mL×2) was performed, and then an organic layer was dried with MgSO₄ and concentrated under reduced pressure. Thereafter, purification was performed with silica gel column chromatography (MeOH:DCM = 1:10) to obtain a title compound which is an orange solid (11 mg, yield: 19%).

¹H NMR (300 MHz, CDCl₃) δ 8.33 (s, 1H), 8.04 (s, 1H), 7.30-7.27 (m, 1H), 7.25-7.24 (m, 2H), 7.20-7.15 (m, 7H), 7.08-7.00 (m, 3), 6.74 (dd, *J* = 8.6, 2.3 Hz, 4H), 5.95 (d, *J* = 6.0 Hz, 1H), 5.48 (s, 2), 4.73 (t, *J* = 5.6 Hz, 1H), 4.46 (s, 1H), 4.36 (d, *J* = 4.8 Hz, 1H), 3.81 (s, 2H), 3.75 (s, 6H), 3.48 (s, 3H), 3.44 (dd, *J* = 10.8, 3.5 Hz, 1H), 3.22 (dd, *J* = 10.4, 3.3 Hz, 1H).

[Example 26] Preparation of (2R,3S,4R,5R)-2-(((tert-butyldiphenylsilyl)oxy)methyl)-5-(6-chloro-9H-purin-9-yl)tetrahydrofuran-3,4-diol (Compound 26)

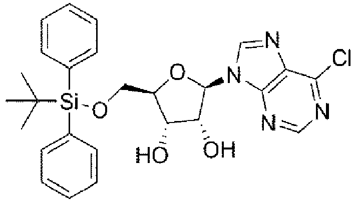

6-Chloropurine riboside (100 mg, 0.348 mmol) was dissolved in DMF (4 mL), DIPEA (151 µL, 0.872 mmol), DMAP (4 mg, 0.0348 mmol), and tert-butyldiphenylchlorosilane (TBDPSCl, 99 µL, 0.383 mmol) were added, and stirring was performed at room temperature for 3 hours. The reaction was completed by adding H₂O and then extraction with EA was performed. An organic layer was washed with aqueous NaHCO₃ and NH₄Cl solutions. The obtained organic layer was dried with Na₂SO₄ and filtered. The filtrate was concentrated under reduced pressure and purified with silica gel column chromatography to obtain a title compound which is a white solid (150 mg, yield: 81%).

¹H NMR (400 MHz, CDCl₃) δ 8.68 (s, 1H), 8.39 (s, 1H), 7.59-7.51 (m, 4H), 7.45-7.38 (m, 2H), 7.37-7.28 (m, 4H), 6.06 (d, *J* = 5.5 Hz, 1H), 5.04 (d, *J* = 3.8 Hz, 1H), 4.79-4.72 (m, 1H), 4.58-4.52 (m, 1H), 4.36 (q, *J* = 3.1 Hz, 1H), 3.95 (dd, *J* = 11.6, 3.4 Hz, 1H), 3.85 (dd, *J* = 11.6, 3.0 Hz, 1H), 3.21 (dd, *J* = 4.9, 2.8 Hz, 1H), 0.94 (s, 9H).

[Example 27] Preparation of (2R,3S,4R,5R)-2-(((tert-butyldiphenylsilyl)oxy)methyl)-5-(6-(1-methyl-1H-indol-2-yl)-9H-purin-9-yl)tetrahydrofuran-3,4-diol (Compound 27)

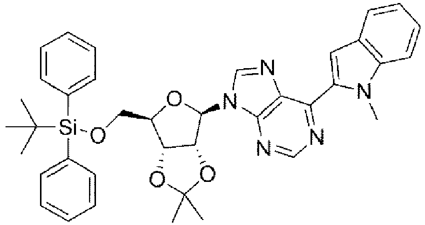

To (2R,3S,4R,5R)-2-(((tert-butyldiphenylsilyl)oxy)methyl)-5-(6-chloro-9H-purin-9-yl)tetrahydrofuran-3,4-diol (19 mg, 0.0336 mmol), Pd(PPh₃)₄ (3.8 mg, 0.00336 mmol), CsCO₃ (32 mg, 0.100 mmol), and 1-methyl-2-indoleboronic acid pinacol ester (17 mg, 0.067 mmol) were added, the reactants were dissolved in 1,4-dioxane:H₂O=5:1 (2.5 mL), and then stirring was performed at 100°C for 10 hours. The reaction mixture was cooled to room temperature and then concentrated under reduced pressure to remove the solvent. After dilution with EA, H₂O was added thereto to perform extraction. An organic layer was dried with Na₂SO₄ and then filtered. The filtrate was concentrated under reduced pressure and purified with column chromatography to obtain a coupled compound as a colorless liquid (19 mg, 86%). The coupled compound (8 mg, 0.0121 mmol) was dissolved in CH₂Cl₂ (3 mL), TFA (1 mL) and H₂O (0.5 mL) were added thereto, and stirring was performed at room temperature for 6 hours. The reaction mixture was concentrated under reduced pressure and purified with silica gel column chromatography to obtain a title compound as a yellow liquid (2 mg, yield: 29%).

¹H NMR (400 MHz, Chloroform-*d*) δ 8.91 (s, 1H), 8.37 (s, 1H), 8.12 (s, 1H), 7.76 (d, *J* = 7.9 Hz, 1H), 7.61-7.51 (m, 4H), 7.46 (d, J = 8.4 Hz, 1H), 7.44-7.28 (m, 8H), 7.19-7.12 (m, 1H), 6.05 (d, *J* = 6.1 Hz, 1H), 4.78 (t, *J* = 5.6 Hz, 1H), 4.55 (d, *J* = 5.2 Hz, 1H), 4.46-4.41 (m, 1H), 4.33 (s, 3H), 3.92 (dd, *J* = 11.5, 3.5 Hz, 1H), 3.84 (dd, *J* = 11.5, 3.0 Hz, 1H), 3.02 (s, 1H), 0.91 (s, 9H).

[Example 28] Preparation of (2R,3S,4R,5S)-2-(((tert-butyldiphenylsilyl)oxy)methyl)-5-(6-((3-ethynylphenyl)amino)-9H-purin-9-yl)tetrahydrofuran-3,4-diol

### (Compound 28)

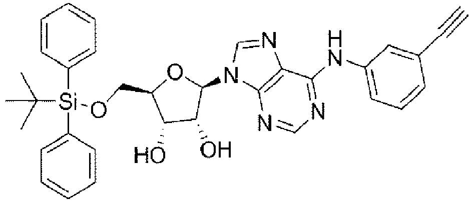

(2*R*,3S,4R,5R)-2-(((tert-butyldiphenylsilyl)oxy)methyl)-5-(6-chloro-9H-purin-9-yl)tetrahydrofuran-3,4-diol (17 mg, 0.0323 mmol) was dissolved in n-BuOH (3 mL), DIPEA (22 µL, 0.129 mmol) and 3-ethynylaniline (7 µL, 0.0647 mmol) were added thereto, and stirring was performed at 130°C for 1.5 days. The reaction mixture was cooled to room temperature and then concentrated under reduced pressure to remove the solvent. After dilution with EA, H₂O was added thereto to perform extraction. An organic layer was washed with aqueous NaHCO₃ and NH₄Cl solutions. The organic layer was dried with Na₂SO₄ and then filtered. The filtrate was concentrated under reduced pressure and purified with silica gel column chromatography to obtain a title compound which is a brown solid (12 mg, yield: 63%).

¹H NMR (300 MHz, CDCl₃) δ 8.48 (s, 1H), 8.11 (s, 1H), 8.03 (s, 1H), 7.84-7.74 (m, 2H), 7.59-7.50 (m, 4H), 7.45-7.26 (m, 8H), 6.39 (s, 1H), 5.98 (d, *J* = 6.2 Hz, 1H), 4.71 (t, *J* = 5.6 Hz, 1H), 4.52 (dd, *J* = 4.9, 1.5 Hz, 1H), 4.45-4.39 (m, 1H), 3.89 (dd, *J* = 11.5, 3.4 Hz, 1H), 3.82 (dd, *J* = 11.5, 2.9 Hz, 1H), 3.23 (s, 1H), 3.11 (s, 1H), 0.91 (s, 9H) .

[Example 29] Preparation of 9-((3a*R*, 4R,6R,6aR)-6-(((tert-butyldiphenylsilyl)oxy)methyl)-2,2-dimethyltetrahydrofuro[3,4-d][1,3]dioxol-4-yl)-2-chloro-N-cyclopentyl-9H-purin-6-amine (Compound 29)

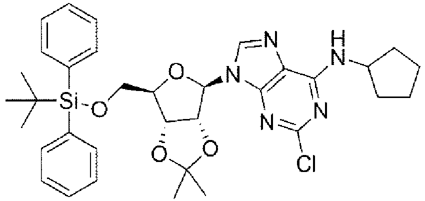

((3a*R*, 4R,6R,6a*R*)-6-(2-chloro-6-(cyclopentylamino) -9H-purin-9-yl)-2,2-dimethyltetrahydrofuro[3,4-d] [1,3]dioxol-4-yl)methanol (41.0 mg, 0.1 mmol) and 4-dimethylaminopyridine (1.0 mg, 0.1 eq, 0.01 mmol) were added to a 7 mL vial and were dissolved in pyridine (0.225 M, 0.44 mL). Then, *tert-*butylchlorodiphenylsiliane (0.03 mL, 1.1 eq, 0.11 mmol) was added thereto, and the reactants were reacted at room temperature for 4 hours. After completion of the reaction, extraction with NaHCO₃ (20 mL) and ethyl acetate (25 mL×2) was performed, and then an organic layer was dried with MgSO₄ and concentrated under reduced pressure. Thereafter, purification was performed with silica gel column chromatography (EA:Hex = 1:1) to obtain a title compound which is a white solid (61.1 mg, 94.2%).

¹H NMR (500 MHz, CDCl₃) δ 7.81 (s, 1H), 7.63-7.60 (m, 2H), 7.59-7.55 (m, 2H), 7.42-7.27 (m, 6H), 6.06 (d, *J* = 2.7 Hz, 1H), 5.24 (dd, *J* = 6.3, 2.7 Hz, 1H), 4.99 (dd, *J* = 6.4, 3.2 Hz, 1H), 4.36 (td, *J* = 5.1, 3.3 Hz, 1H), 3.90 (dd, *J* = 11.2, 4.8 Hz, 1H), 3.80 (dd, *J* = 11.1, 5.5 Hz, 1H), 2.14 (dt, *J* = 12.7, 7.1 Hz, 2H), 1.82-1.65 (m, 4H), 1.61 (s, 3H), 1.38 (s, 3H), 1.04 (s, 9H).

[Example 30] Preparation of (2R,3S,4R,5R)-2-(2,2-diphenyl-2-(pyridin-4-yl)ethyl)-5-(6-((3-fluorobenzyl)(methyl)amino)-9H-purin-9-yl)tetrahydrofuran-3,4-diol (Compound 30)

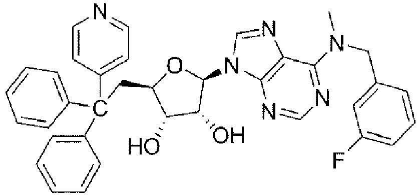

Compound 30 was synthesized using the method of the above Example. LC/MS (M+H): 617

[Example 31] Preparation of (2R,3S,4R,5R)-2-(2,2-diphenyl-2-(pyridin-4-yl)ethyl)-5-(6-((3-fluorobenzyl)(methyl)amino)-9H-purin-9-yl)tetrahydrofuran-3,4-diol (Compound 31)

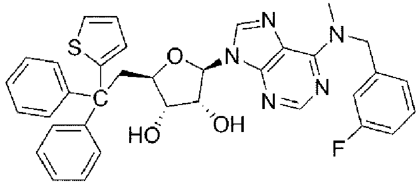

Compound 31 was synthesized using the method of the above Example. LC/MS (M+H): 622

[Example 32] Preparation of (2R,3R,4S,5R)-2-(6-((3-fluorobenzyl)(methyl)amino)-9H-purin-9-yl)-5-(((5-methylisoxazol-3-yl)methoxy)methyl)tetrahydrofuran-3,4-diol

### (Compound 32)

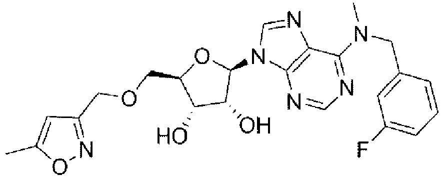

Compound 32 was synthesized using the method of the above Example. LC/MS (M+H): 485

[Example 33] Preparation of (2R,3R,4S,5R)-2-(6-((3-fluorobenzyl)(methyl)amino)-9H-purin-9-yl)-5-(((2-methyl-6-(trifluoromethyl)pyridin-3-yl)methoxy)methyl)tetrahydrofuran-3,4-diol (Compound 33)

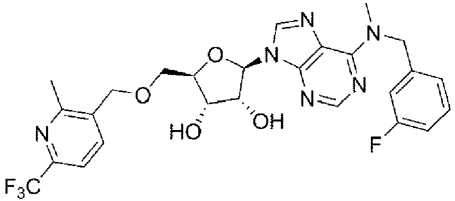

Compound 33 was synthesized using the method of the above Example. LC/MS (M+H): 563

[Example 34] Preparation of (2R,3S,4R,5R)-2-(((6-(1H-pyrazol-1-yl)pyridin-3-yl)methoxy)methyl)-5-(6-((3-fluorobenzyl)(methyl)amino)-9H-purin-9-yl)tetrahydrofuran-3,4-diol (Compound 34)

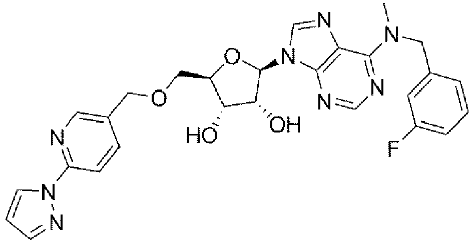

Compound 34 was synthesized using the method of the above Example. LC/MS (M+H): 547

[Example 35] Preparation of (2R,3R,4S,5R)-2-(6-((3-fluorobenzyl)(methyl)amino)-9H-purin-9-yl)-5-((quinolin-2-ylmethoxy)methyl)tetrahydrofuran-3,4-diol (Compound 35)

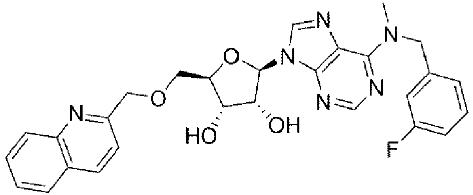

Compound 35 was synthesized using the method of the above Example. LC/MS (M+H): 531

[Example 36] Preparation of (2R,3S,4R,5R)-2-(((1H-pyrrolo[2,3-b]pyridin-5-yl)methoxy)methyl)-5-(6-((3-fluorobenzyl)(methyl)amino)-9H-purin-9-yl)tetrahydrofuran-3,4-diol (Compound 36)

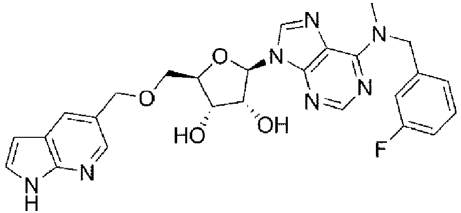

Compound 36 was synthesized using the method of the above Example. LC/MS (M+H): 520

[Example 37] Preparation of ((2R,3S,4R,5R)-5-(6-((3-fluorobenzyl)(methyl)amino)-9H-purin-9-yl)-3,4-dihydroxytetrahydrofuran-2-yl)methyl pyridin-3-ylcarbamate

### (Compound 37)

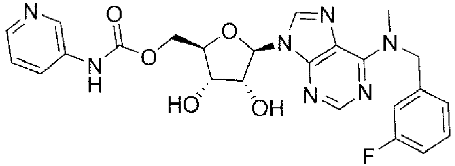

Compound 37 was synthesized using the method of the above Example. LC/MS (M+H): 510

[Example 38] Preparation of ((2R,3S,4R,5R)-5-(6-((3-fluorobenzyl)(methyl)amino)-9H-purin-9-yl)-3,4-dihydroxytetrahydrofuran-2-yl)methyl benzo[d][1,3]dioxol-5-ylcarbamate (Compound 38)

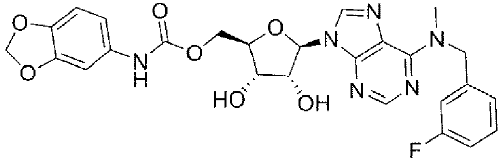

Compound 38 was synthesized using the method of the above Example. LC/MS (M+H): 553

[Example 39] Preparation of ((2R,3S,4R,5R)-5-(6-((3-fluorobenzyl)(methyl)amino)-9H-purin-9-yl)-3,4-dihydroxytetrahydrofuran-2-yl)methyl (5-methyl-3-phenylisoxazol-4-yl)carbamate (Compound 39)

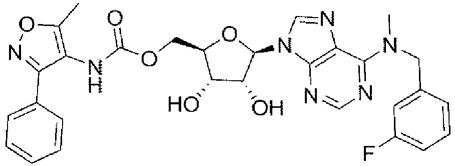

Compound 43 was synthesized using the method of the above Example. LC/MS (M+H): 590

[Example 40] Preparation of O-(((2R,3S,4R,5R)-5-(6-((3-fluorobenzyl)(methyl)amino)-9H-purin-9-yl)-3,4-dihydroxytetrahydrofuran-2-yl)methyl) (5-bromoquinoxalin-6-yl)carbamothioate (Compound 40)

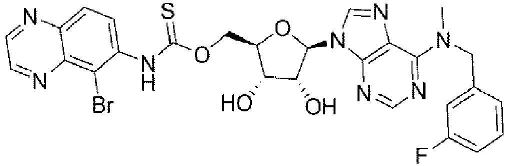

Compound 40 was synthesized using the method of the above Example. LC/MS (M+H): 655

[Example 41] Preparation of O-(((2R,3S,4R,5R)-5-(6-((3-fluorobenzyl)(methyl)amino)-9H-purin-9-yl)-3,4-dihydroxytetrahydrofuran-2-yl)methyl) (5-bromoquinoxalin-6-yl)carbamothioate (Compound 41)

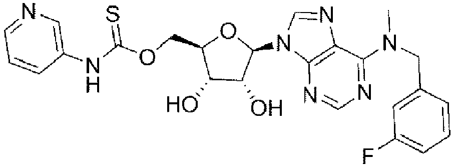

Compound 41 was synthesized using the method of the above Example. LC/MS (M+H): 526

[Example 42] Preparation of O-(((2R,3S,4R,5R)-5-(6-((3-fluorobenzyl)(methyl)amino)-9H-purin-9-yl)-3,4-dihydroxytetrahydrofuran-2-yl)methyl) benzo[d][1,3]dioxol-5-ylcarbamothioate (Compound 42)

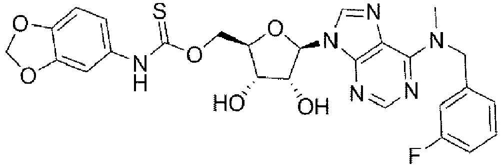

Compound 46 was synthesized using the method of the above Example. LC/MS (M+H): 569

[Example 43] Preparation of (2R,3S,5R)-2-(((tert-butyldiphenylsilyl)oxy)methyl)-5-(6-((3-fluorobenzyl)(methyl)amino)-9H-purin-9-yl)tetrahydrofuran-3-ol (Compound 43)

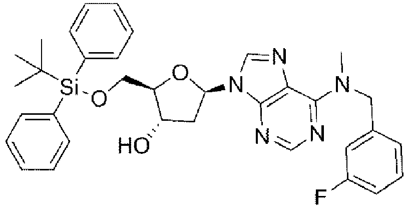

Compound 43 was synthesized using the method of the above Example. LC/MS (M+H): 612

[Example 44] Preparation of (2R,3R,4S,5R)-2-(((tert-butyldiphenylsilyl)oxy)methyl)-4-fluoro-5-(6-((3-fluorobenzyl)(methyl)amino)-9H-purin-9-yl)tetrahydrofuran-3-ol (Compound 44)

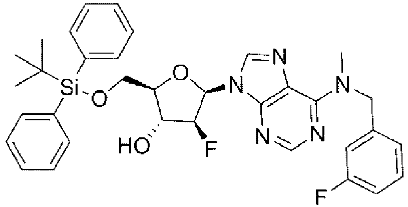

Compound 44 was synthesized using the method of the above Example. LC/MS (M+H): 630

[Example 45] Preparation of (2R,3R,4R,5R)-2-(((tert-butyldiphenylsilyl)oxy)methyl)-4-fluoro-5-(6-((3-fluorobenzyl)(methyl)amino)-9H-purin-9-yl)tetrahydrofuran-3-ol (Compound 45)

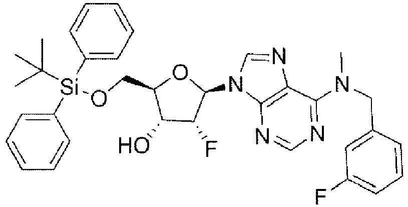

Compound 45 was synthesized using the method of the above Example. LC/MS (M+H): 630

[Example 46] Preparation of (2R,3R,4R,5R)-2-(((tert-butyldiphenylsilyl)oxy)methyl)-4-fluoro-5-(6-((3-fluorobenzyl)(methyl)amino)-9H-purin-9-yl)tetrahydrofuran-3-ol (Compound 46)

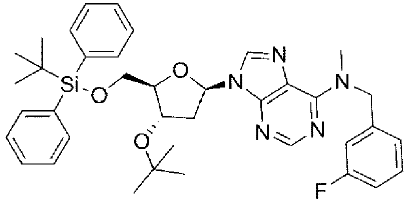

Compound 46 was synthesized using the method of the above Example. LC/MS (M+H): 668

<Experimental Example 1> Enzyme assay

Referring to a method described in a reference document [Front Pharmacol. 2018 Mar 1;9:153], an enzyme assay was performed. Each of human CD73 and CD39 recombinant proteins was produced and then purified and isolated, and used in an enzyme assay. A buffer was basically 25 mM Tris, 5 mM MgCl₂, pH 7.5 as an assay buffer, and for assay, human CD73 and CD39 recombinant protein were diluted at an appropriate concentration, respectively, in the assay buffer, adenosine monophosphate (AMP) (Sigma, Catalog # A1752) at an appropriate concentration was added, and then incubation was performed for 30 minutes. At the time, the azolopyrimidine heterocyclic compounds of the present invention were treated for each concentration and incubated together, thereby measuring a degree of inhibiting activity of CD73 or CD39. The activity of the corresponding protein was measured with Malachite green kit, and the results are shown in the following Table 1:

**[Table 1]**

| | Enzyme Activity (IC₅₀, µM) | | | Enzyme Activity (IC₅₀, µM) | |
|---|---|---|---|---|---|
| | CD39 | CD73 | | CD39 | CD73 |
| Compound 1 | - | E | Compound 16 | C | B |
| Compound 2 | - | B | Compound 17 | B | B |
| Compound 3 | - | D | Compound 18 | D | B |
| Compound 4 | - | D | Compound 19 | B | B |
| Compound 6 | - | C | Compound 20 | B | B |
| Compound 7 | E | B | Compound 21 | B | A |
| Compound 8 | B | A | Compound 22 | C | A |
| Compound 9 | - | C | Compound 23 | E | B |
| Compound 13 | B | A | Compound 24 | B | A |
| Compound 14 | C | A | Compound 25 | A | A |
| Compound 15 | B | B | | | |
| A: 0-1.0 µM; B: 1.1-5.0 µM; C: 5.1-15.0 µM; D: 15.1-30.0 µM; | | | | | |
| E: 30 µM or more; -: not measured. | | | | | |

As described above, the compound according to the present invention may inhibit the enzyme activity of CD73 and CD39 to prevent conversion from ATP into adenosine, thereby preventing a cancer tissue-specific immunity lowering mechanism. Accordingly, the compound of the present invention may be useful as the effective component of the pharmaceutical composition for prophylaxis or treatment of cancer.

## Claims

1. A pharmaceutical composition for prophylaxis or treatment of cancer comprising: an azolopyrimidine heterocyclic compound represented by the following Chemical Formula 1, a prodrug thereof, a hydrate thereof, a solvate thereof, a stereoisomer thereof, or a pharmaceutically acceptable salt thereof as an effective component: 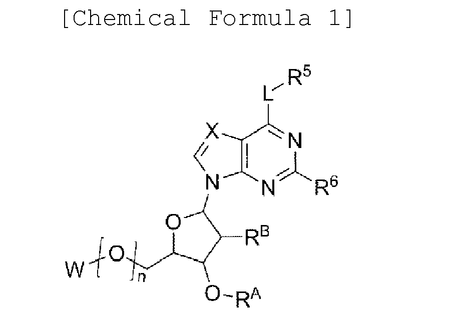 wherein
R^{A} is hydrogen, C1-C20alkyl, or acetyl;
R^{B} is hydrogen, a halogen, or -O-R^{c};
R^{c} is hydrogen, C1-C20alkyl, or acetyl, or the R^{c} may be linked to R^{A} via C1-C20alkylene to form a fused ring;
X is CR' or N;
R' is hydrogen, C1-C20alkyl, or C3-C20cycloalkyl;
L is a single bond, NR", O, S, or S=O;
R" is hydrogen, C1-C20alkyl, or C3-C20cycloalkyl;
n is an integer of 0 or 1;
W is 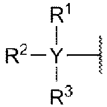 or 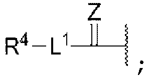
Y is C or Si;
R¹ is C1-C20alkyl, C6-C20aryl, C2-C20heteroaryl, or C2-C20heterocycloalkyl;
R² and R³ are independently of each other hydrogen, C1-C20alkyl, C6-C20aryl, C2-C20heteroaryl, or C2-C20heterocycloalkyl, or R² and R³ may be linked via C1-C20alkaylene to form a fused ring;
Z is O or S;
L¹ is a single bond, NR''', O, or S;
R''' is hydrogen, C1-C20alkyl, or C3-C20cycloalkyl;
R⁴ is C6-C20aryl, C3-C20cycloalkyl, C2-C20heteroaryl, or C2-C20heterocycloalkyl;
R⁵ is hydrogen, a halogen, C1-C20alkyl, -(CH₂)ₘ-Ar¹, C6-C20aryl, C3-C20cycloalkyl, C2-C20heteroaryl, or C2-C20heterocycloalkyl;
m is an integer of 1 to 10;
Ar¹ is C6-C20aryl, C3-C20cycloalkyl, C2-C20heteroaryl, or C2-C20heterocycloalkyl;
R⁶ is hydrogen, a halogen, or NR^{a}R^{b};
R^{a} and R^{b} are independently of each other hydrogen, C1-C20alkyl, C3-C20cycloalkyl, or C6-C20aryl;
the alkyl, aryl, heteroaryl, or heterocycloalkyl of R¹ to R³, the alkyl, aryl, cycloalkyl, heteroaryl, or heterocycloalkyl of R⁵, the aryl, cycloalkyl, heteroaryl, or heterocycloalkyl of R⁴ and Ar¹, or the alkyl or aryl of R^{a} and R^{b} may be further substituted by one or more selected from a halogen, C1-C20alkyl, haloC1-C20alkyl, C1-C20alkoxy, haloC1-C20alkoxy, C6-C20aryl, C6-C20aryloxy, C1-C20alkylsulfanyl, haloC1-C20alkylsulfanyl, C3-C20cycloalkylsulfanyl, C6-C20arylsulfanyl, pentafluorosulfanyl, pentafluorosulfanyloxy, C3-C20cycloalkyl, C2-C20heteroaryl, C2-C20alkenyl, C2-C20alkynyl, amino, C1-C20alkylamino, C6-C20arylamino, hydroxy, C1-C20alkylcarbonyl, C1-C20alkoxycarbonyl, C1-C20alkylcarbonyloxy, C1-C20alkoxycarbonyloxy, nitro, mercapto, formyl, carboxyl, sulfamoyl, and carbamoyl; and
the heteroaryl and the heterocycloalkyl contain one or more heteroatom selected from nitrogen, oxygen, and sulfur.

2. The pharmaceutical composition for prophylaxis or treatment of cancer of claim 1, wherein the compound of Chemical Formula 1 is represented by the following Chemical Formula 2, Chemical Formula 3, or Chemical Formula 4: 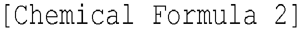 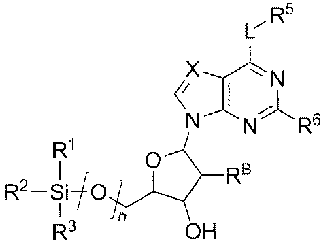 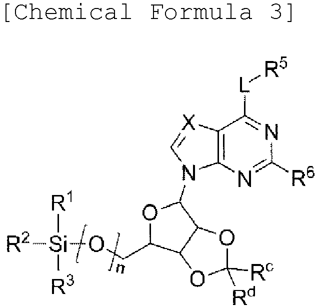 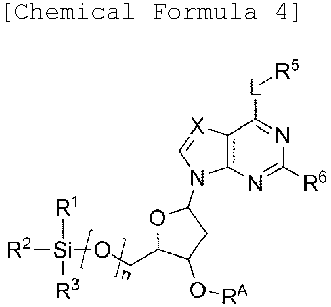
wherein X, L, R¹ to R³, R⁵, and R⁶ are as defined in Chemical Formula 1 of claim 1;
n is an integer of 0 or 1;
R^{B} is hydrogen, a halogen, or hydroxy;
R^{A} is C1-C10alkyl; and
R^{c} and R^{d} are independently of each other hydrogen or C1-C10alkyl.

3. The pharmaceutical composition for prophylaxis or treatment of cancer of claim 2, wherein in Chemical Formulae 2 to 4,
R¹ is C1-C10alkyl, C6-C12aryl, C2-C12heteroaryl, or C2-C10heterocycloalkyl; R² and R³ are independently of each other C1-C10alkyl, C6-C12aryl, C2-C12heteroaryl, or C2-C10heterocycloalkyl, or R² and R³ may be linked via C1-C10alkylene to form a fused ring; L is a single bond, NR", O, S, or S=O; R" is hydrogen or C1-C10alkyl; R⁵ is hydrogen, a halogen, C1-C10alkyl, -(CH₂)ₘ-Ar¹, C6-C12aryl, C3-C10cycloalkyl, C2-C12heteroaryl, or C2-C10heterocycloalkyl;
m is an integer of 1 to 7; Ar¹ is C6-C12aryl, C3-C10cycloalkyl, C2-C12heteroaryl, or C2-C10heterocycloalkyl;
R⁶ is hydrogen or halogen; and the alkyl, aryl, heteroaryl,
or heterocycloalkyl of R¹ to R³, the alkyl, aryl, cycloalkyl, heteroaryl, or heterocycloalkyl of R⁵, and the aryl, cycloalkyl, heteroaryl, or heterocycloalkyl of Ar¹ may be further substituted by one or more selected from a halogen, C1-C10alkyl, haloC1-C10alkyl, C1-C10alkoxy, haloC1-C10alkoxy, C6-C12aryl, C6-C12aryloxy, C1-C10alkylsulfanyl, haloC1-C10alkylsulfanyl, C3-C10cycloalkylsulfanyl, C6-C12arylsulfanyl, pentafluorosulfanyl, pentafluorosulfanyloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl.

4. The pharmaceutical composition for prophylaxis or treatment of cancer of claim 1, wherein the compound of Chemical Formula 1 is represented by the following Chemical Formula 5, Chemical Formula 6, or Chemical Formula 7: 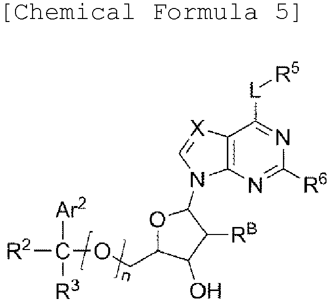 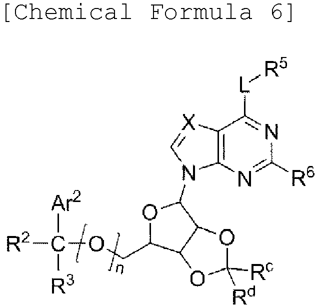 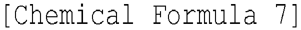 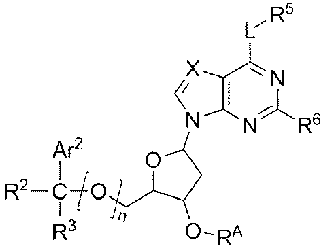
wherein X, L, R⁵, and R⁶ are as defined in Chemical Formula 1 of claim 1;
n is an integer of 0 or 1;
R^{B} is hydrogen, a halogen, or hydroxy;
R^{A} is C1-C10alkyl; and
R^{c} and R^{d} are independently of each other hydrogen or C1-C10alkyl;
Ar² is C6-C12aryl or C2-C12heteroaryl;
R² and R³ are independently of each other hydrogen or C6-C12aryl; and
the aryl of Ar² or the aryl of R² and R³ may be further substituted by one or more selected from a halogen, C1-C10alkyl, C1-C10alkoxy, C6-C12aryl, C6-C12aryloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl, and the heteroaryl of Ar² may be further substituted by one or more selected from a halogen, C1-C10alkyl, haloC1-C10alkyl, C1-C10alkoxy, C6-C12aryl, C6-C12aryloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl.

5. The pharmaceutical composition for prophylaxis or treatment of cancer of claim 1, wherein the compound of Chemical Formula 1 is represented by the following Chemical Formula 8, Chemical Formula 9, or Chemical Formula 10: 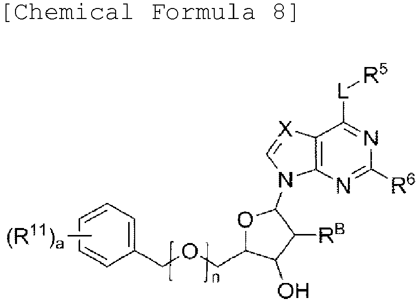 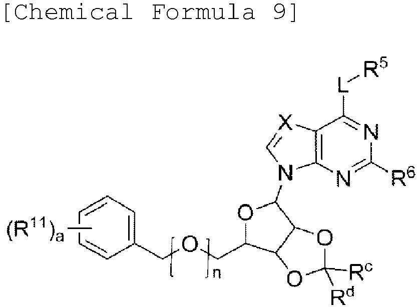 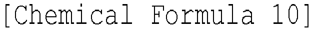 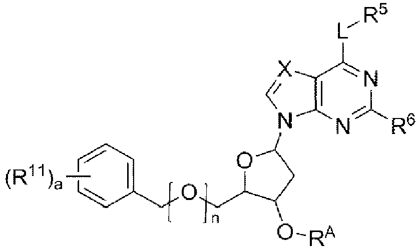
wherein X, L, R⁵, and R⁶ are as defined in Chemical Formula 1 of claim 1;
n is an integer of 0 or 1;
R^{B} is hydrogen, a halogen, or hydroxy;
R^{A} is C1-C10alkyl; and
R^{c} and R^{d} are independently of each other hydrogen or C1-C10alkyl;
R¹¹ is haloC1-C10alkyl or pentafluorosulfanyl; and
a is an integer of 1 to 5.

6. The pharmaceutical composition for prophylaxis or treatment of cancer of claim 1, wherein the compound of Chemical Formula 1 is represented by the following Chemical Formula 11, Chemical Formula 12, or Chemical Formula 13: 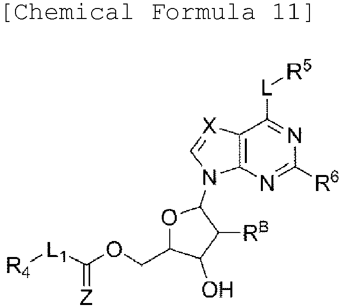 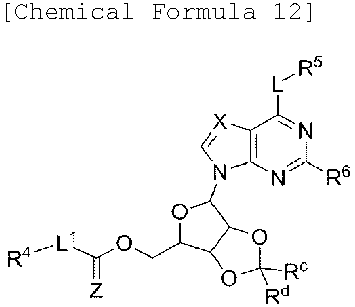 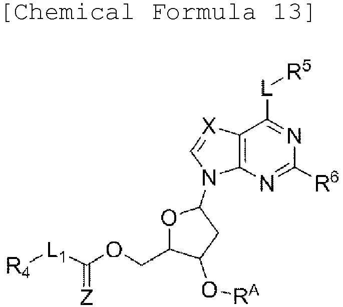
wherein X, L, R⁵, and R⁶ are as defined in Chemical Formula 1 of claim 1;
R^{B} is hydrogen, a halogen, or hydroxy;
R^{A} is C1-C10alkyl; and
R^{c} and R^{d} are independently of each other hydrogen or C1-C10alkyl;
Z is O or S;
L¹ is a single bond, NH, or O;
R⁴ is C6-C12aryl or C2-C12heteroaryl; and
the aryl or heteroaryl of R⁴ may be further substituted by one or more selected from a halogen, C1-C10alkyl, haloC1-C10alkyl, C1-C10alkoxy, C6-C12aryl, C6-C12aryloxy, C3-C10cycloalkyl, C2-C12heteroaryl, C2-C10alkenyl, C2-C10alkynyl, amino, C1-C10alkylamino, C6-C12arylamino, hydroxy, C1-C10alkylcarbonyl, C1-C10alkoxycarbonyl, C1-C10alkylcarbonyloxy, C1-C10alkoxycarbonyloxy, nitro, mercapto, formyl, carboxyl, sulfamoyl, and carbamoyl.

7. The pharmaceutical composition for prophylaxis or treatment of cancer of claim 2, wherein the azolopyrimidine heterocyclic compound is any one selected from the following structures, a prodrug thereof, a hydrate thereof, a solvate thereof, a stereoisomer thereof, or a pharmaceutically acceptable salt thereof:

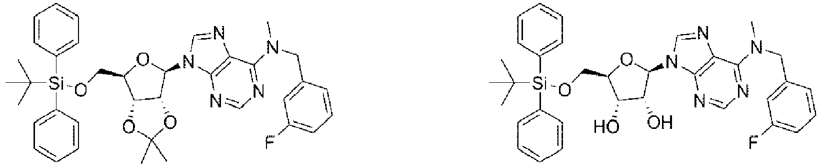

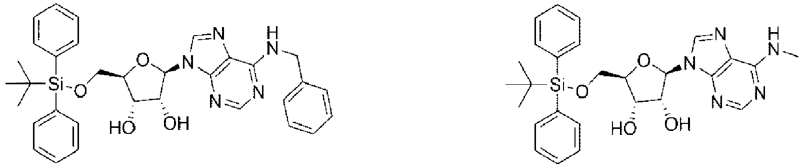

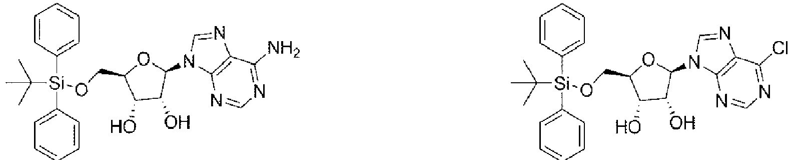

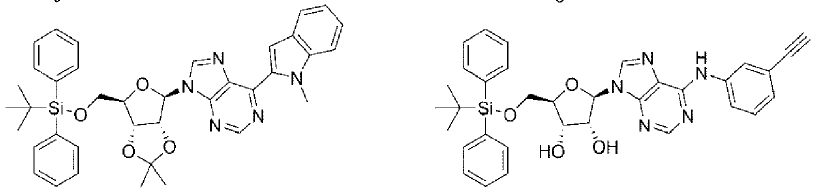

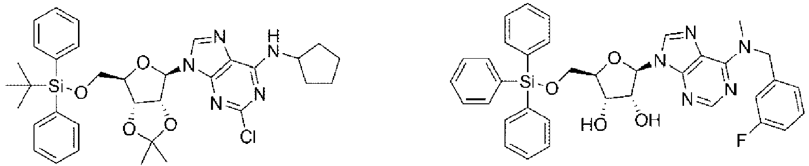

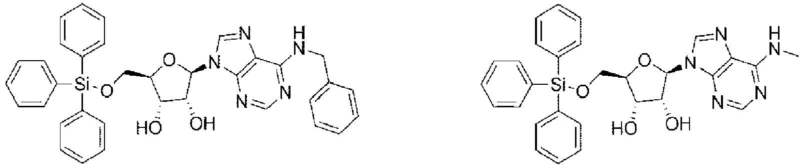

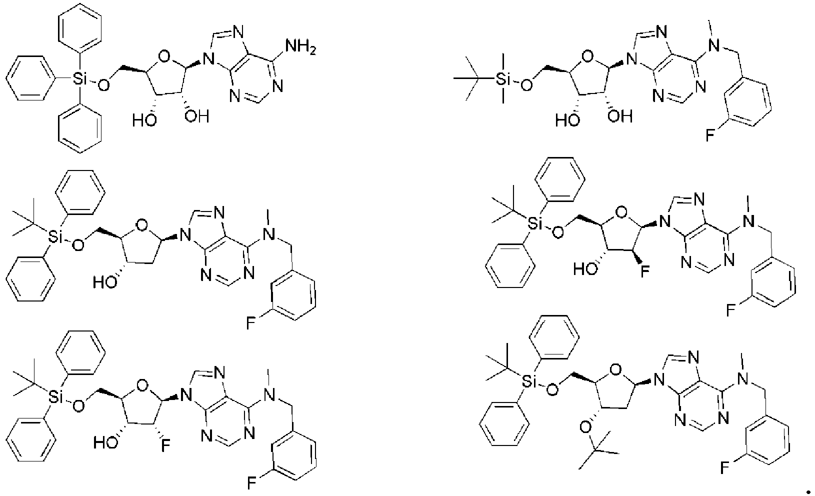

8. The pharmaceutical composition for prophylaxis or treatment of cancer of claim 4, wherein the azolopyrimidine heterocyclic compound is any one selected from the following structures, a prodrug thereof, a hydrate thereof, a solvate thereof, a stereoisomer thereof, or a pharmaceutically acceptable salt thereof:

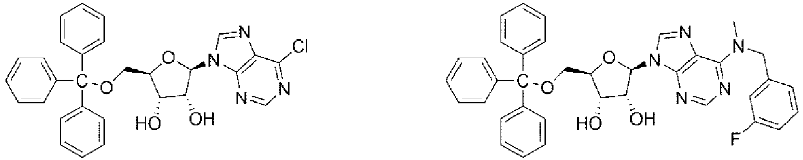

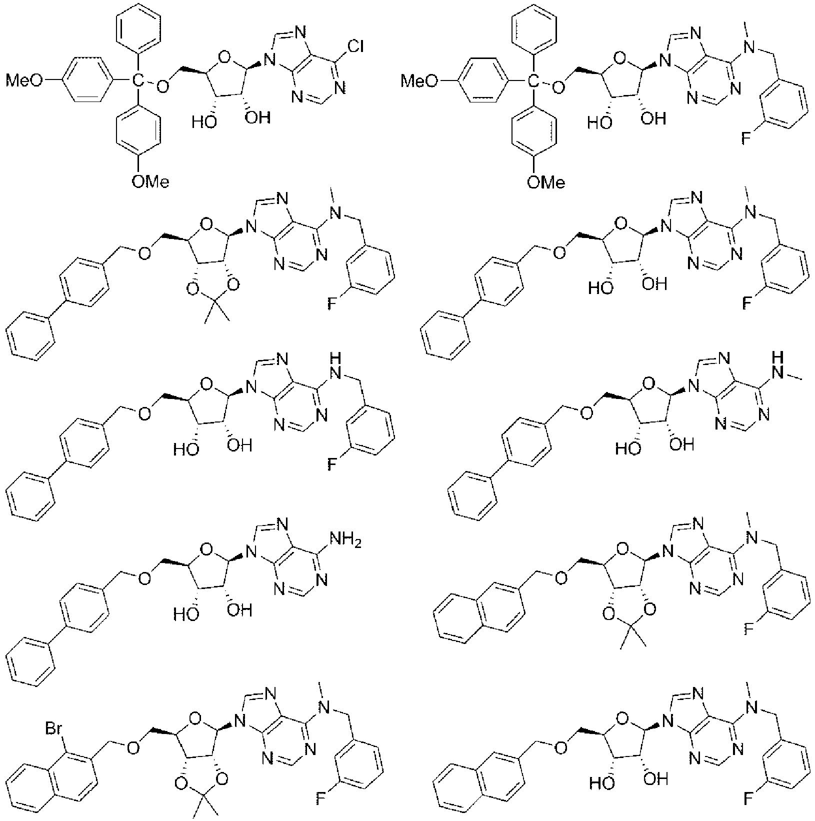

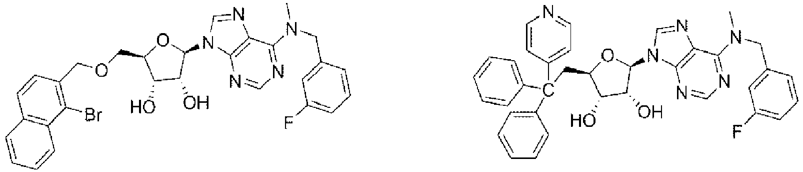

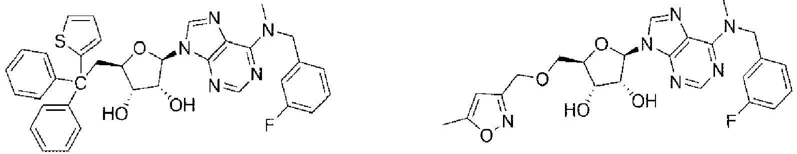

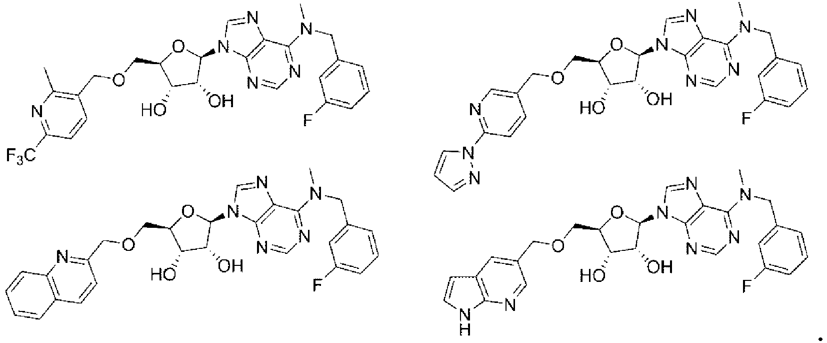

9. The pharmaceutical composition for prophylaxis or treatment of cancer of claim 5, wherein the azolopyrimidine heterocyclic compound is any one selected from the following structures, a prodrug thereof, a hydrate thereof, a solvate thereof, a stereoisomer thereof, or a pharmaceutically acceptable salt thereof:

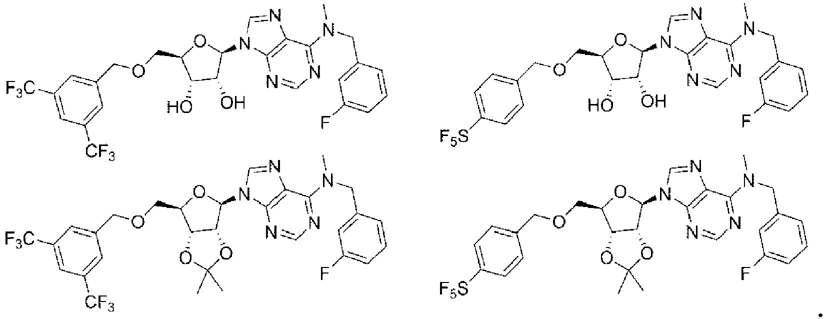

10. The pharmaceutical composition for prophylaxis or treatment of cancer of claim 6, wherein the azolopyrimidine heterocyclic compound is any one selected from the following structures, a prodrug thereof, a hydrate thereof, a solvate thereof, a stereoisomer thereof, or a pharmaceutically acceptable salt thereof:

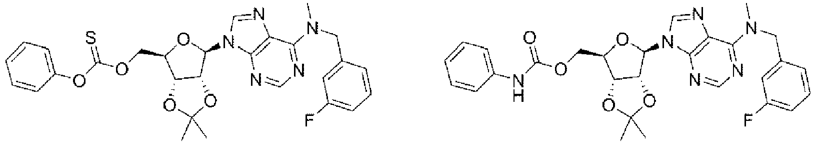

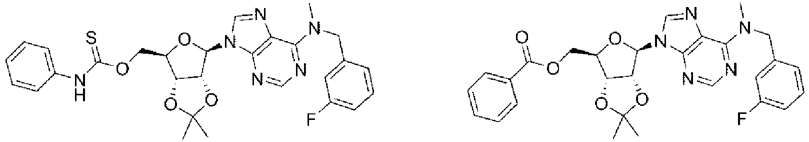

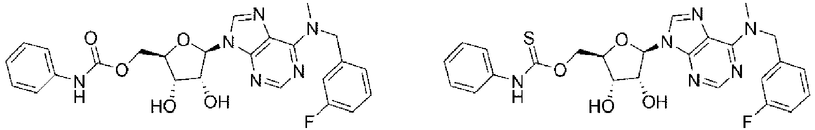

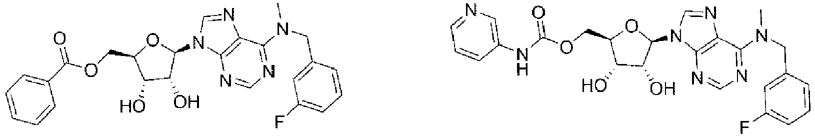

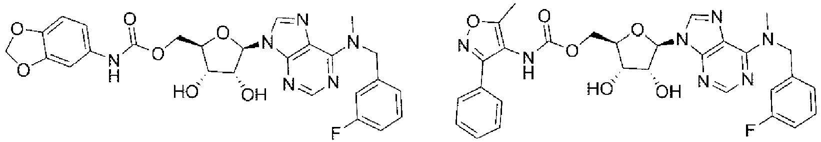

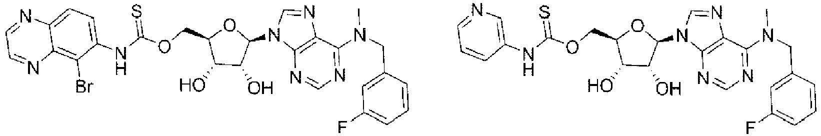

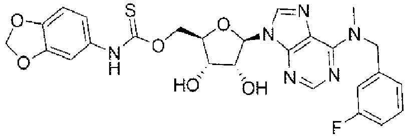

11. The pharmaceutical composition for prophylaxis or treatment of cancer of claim 1, wherein the cancer is liver cancer, colon cancer, prostate cancer, stomach cancer, lung cancer, or breast cancer.

12. The pharmaceutical composition for prophylaxis or treatment of cancer of claim 1, further comprising: a pharmaceutically acceptable carrier, excipient, or diluent.
